# EUROPEAN PATENT SPECIFICATION

(11) **EP 3 739 058 B1**
(45) Date of publication and mention of the grant of the patent: **26.07.2023**
(21) Application number: 20177048.4
(22) Date of filing: 30.03.2016
(51) Int. Cl.: C12Q 1/02, C12Q 1/68, C12Q 1/6895

(54) **NOVEL GENE REGULATING VIRULENCE OF CRYPTOCOCCUS NEOFORMANS, AND USE THEREOF**
NEUARTIGES GEN ZUR REGULIERUNG DER VIRULENZ VON CRYPTOCOCCUS NEOFORMANS UND VERWENDUNG DAVON
NOUVEAU GÈNE RÉGLANT LA VIRULENCE DE CRYPTOCOCCUS NEOFORMANS ET UTILISATION CORRESPONDANTE

(30) Priority: 30.03.2015 KR 20150044621; 30.03.2015 KR 20150044617
(43) Date of publication of application: 18.11.2020
(62) Divisional of application: 16773434.2
(73) Proprietor: Amtixbio Co., Ltd., Seoul 05836 (KR)
(72) Inventor: BAHN, Yong-Sun, Seoul 05538 (KR); JUNG, Kwang-Woo, Seoul (KR); YANG, Dong-Hoon, Namyangju-si, Gyeonggi-do 12109 (KR)
(74) Representative: Steffan & Kiehne Patentanwälte PartG mbB

(56) References cited:
- WO-A2-2010/079998
- WO-A2-2012/087004
- KR-A- 20100 082 579
- ALSPAUGH J A ET AL: "CRYPTOCOCCUS NEOFORMANS MATING AND VIRULENCE ARE REGULATED BY THE G-PROTEIN ALPHA SUBUNIT GPA1 AND CAMP", GENES & DEVELOPMENT, COLD SPRING HARBOR LABORATORY PRESS, PLAINVIEW, NY, US, vol. 11, 1 January 1997 (1997-01-01), pages 3206-3217, XP002952125, ISSN: 0890-9369
- FELICIA J. WALTON ET AL: "Novel gene functions required for melanization of the human pathogen Cryptococcus neoformans : Genes for melanization of C. neoformans", MOLECULAR MICROBIOLOGY, vol. 57, no. 5, 20 July 2005 (2005-07-20), pages 1381-1396, XP055730822, GB ISSN: 0950-382X, DOI: 10.1111/j.1365-2958.2005.04779.x
- DORALYN S DALISAY ET AL: "Ptilomycalin A inhibits laccase and melanization in", BIOORGANIC & MEDICINAL CHEMISTRY, vol. 19, no. 22, 27 May 2011 (2011-05-27), pages 6654-6657, XP028328566, ISSN: 0968-0896, DOI: 10.1016/J.BMC.2011.05.041 [retrieved on 2011-05-27]
- ADA RITA FLORIO ET AL: "Genome-wide expression profiling of the response to short-term exposure to fluconazole in Cryptococcus neoformans serotype A", BMC MICROBIOLOGY, BIOMED CENTRAL LTD, GB, vol. 11, no. 1, 11 May 2011 (2011-05-11), page 97, XP021099691, ISSN: 1471-2180, DOI: 10.1186/1471-2180-11-97
- JIANG NAN ET AL: "A copper-responsive factor gene CUF1 â??is required for copper induction of laccase in Cryptococcus neoformans", FEMS MICROBIOLOGY LETTERS, vol. 296, no. 1, 1 July 2009 (2009-07-01), pages 84-90, XP055873964, ISSN: 0378-1097, DOI: 10.1111/j.1574-6968.2009.01619.x Retrieved from the Internet: URL:https://academic.oup.com/femsle/articl e-pdf/296/1/84/19607553/296-1-84.pdf>

## Description

### [Technical Field]

The present invention relates to novel genes that regulate the virulence of a *Cryptococcus neoformans* strain, and to the use thereof. Moreover, the present invention relates to a method for screening an antifungal agent and a method for screening an agent for treating meningitis, in which the methods comprise measuring the expression of genes that are involved in regulation of the virulence of a *Cryptococcus neoformans* strain.

### [Background Art]

In the past, fungal infections were mainly topical infections such as athlete's foot, jock itch, or oral thrush, and rarely systemic infections. However, recently, systemic infections have frequently occurred such that they accounted for a high frequency of total infections in hospitals.

Antifungal agents developed so far can be largely classified according to chemical structure into two groups: those having an azole structure, and those having no azole structure. The azole-based antifungal agents include ketoconazole, fluconazole, itraconazole, voriconazole and the like, and the non-azole-based antifungal agents include terbinafine, flucytosine, amphotericin B, caspofungin and the like.

Ketoconazole, fluconazole, itraconazole and voriconazole, which have an azole structure, and allylamine-based antifungal agents, such as naftifine and terbinafine, have similar mechanisms of action. These two classes of antifungal agents act to inhibit enzymes required in the process in which lanosterol is converted into ergosterol that is the main component of the fungal cell membrane. The azole-based antifungal agents inhibit microsomal enzymes, and the allylamine-based antifungal agents inhibit squalene epoxidase, thereby exhibiting the above-described effect. Flucytocin (5-FC) is a metabolic antagonist that inhibits nucleic acid synthesis, exhibits antifungal activity by non-competitively antagonizing the overlapping coding of fungal RNA and DNA synthesis. Amphotericin B having a polyene structure exhibits antifungal activity by binding to ergosterol in the fungal cell membrane to induce depolarization of the cell membrane and forming a hole to induce loss of intracellular inclusions. Caspofungin, an echinocandin-based antifungal agent, has an activity of reversibly inhibiting fungal cell wall formation, and differs from the above-mentioned antifungal agents, which act on the cell membrane, in that it acts on the cell wall. The azole-based drug, when administered to patients with reduced liver function, may cause death by hepatitis, and for this reason, a liver function test should precede administration of the azole-based drug. It was reported that flutocytosin has dose-dependent bone marrow suppression and liver toxicity, and can cause enterocolitis. Such side effects further increase when renal function is reduced, and for this reason, monitoring of renal function in patients is very important. In addition, flutocytosin is contraindicated for pregnant women. The major toxicity of amphotericin B is glomerular nephrotoxicity resulting from renal artery vasoconstriction, which is dose-dependent. Thus, when the total cumulative dose of amphotericin B is 4 to 5 g, the possibility of permanent renal function impairment will increase. Furthermore, nephrotoxicity, including the excessive loss of potassium, magnesium and bicarbonate caused by renal tubular toxicity, and a decrease in erythropoietin production, may occur. In addition, as acute responses, symptoms, including thrombophlebitis, rigor, tremor and hyperventilation, may appear.

Meanwhile, *Cryptococcus neoformans* is a basidiomycete fungal pathogen that causes meningoencephalitis in immunocompromised populations, and is responsible for more than 600,000 deaths annually worldwide (Non-Patent Document 1). However, limited therapeutic options are available for treating cryptococcosis (Non-Patent Document 2). Thus, a complete understanding of diverse biological features of Cryptococcus is urgently required for developing novel therapeutic targets and methods. To this end, the signaling cascades governing the general biological features and pathogenicity of *Cryptococcus neoformans* have been extensively studied over the past decades. This study has made it possible to understand several key metabolic and signaling pathways in *Cryptococcus neoformans,* including those involving Ras, cAMP/protein kinase A, Rim101, calmodulin/calcineurin, three MAPKs (Cpk1, Mpk1 and Hog1), the unfolded protein response (UPR), and iron/copper uptake (Non-Patent Document 3 , Non-Patent Document 4 , and Non-Patent Document 5). The present inventors have found that impairment of function of Irel and Hxl1 (HAC1 and XBP1-like gene) proteins, newly identified in Cryptococcus neoformans, and genes encoding the proteins, shows an antifungal effect or a meningitis-treating effect (Patent Document 1 and Patent Document 2). Most of known signaling cascades are composed of sensor/receptor-like proteins and kinases/phosphatases, and are often equipped with unique adaptor or scaffolding proteins to enhance the specificity of each signaling pathway to prevent aberrant crosstalk between the signaling pathways. Nevertheless, each signaling cascade ultimately activates or represses a single transcription factor (TF) or multiple transcription factors, thereby up-regulating or down-regulating effector proteins of transcription factor through transcription factor binding to a specific region of promoter in a target gene that regulates the transcription level of transcription factor. Thus, transcription factor is regarded as a major regulator of gene expression in a given signaling pathway. Particularly, repertoires of transcription factors are often more divergent among species than are those of other signaling components. This appears particularly true in the case of C. *neoformans,* as described in the results of recent genome analyses (Non-Patent Document 6). For example, the UPR signaling pathway which is important in endoplasmic reticulum (ER) stress responses and *Cryptococcus neoformans* virulence is composed of evolutionarily highly conserved Ire1 kinase and Hxl1 transcription factor downstream of the Ire1 kinase. Therefore, *C. neoformans* appears to possess numerous evolutionarily conserved signaling cascades featuring divergent sets of TFs, which might govern the characteristics of C. *neoformans* that are unique compared with those of other fungi.

To understand *C. neoformans* transcription factor networks on a global scale, the present inventors constructed a high-quality gene-deletion mutant through homologous recombination methods for 155 putative *C. neoformans* transcription factors previously predicted, using a DNA-binding domain (DBD) transcription factor database to identify sequence-specific DNA-binding transcription factors in organisms whose genome sequences were analyzed (Non-Patent Document 7 and Non-Patent Document 8). The constructed transcription factor knockout (TFKO) strains were analyzed for 30 distinct *in vitro* phenotypic traits, which cover growth, differentiation, stress responses, antifungal resistance and virulence-factor production. Moreover, the present inventors performed a large-scale virulence test using an insect host model and signature-tagged mutagenesis (STM) scoring in a murine host model, and thus analyzed phenotypes resulting from deletion of various transcription factors in *Cryptococcus neoformans* strains, thereby constructing a comprehensive phenotypic data set (phenome) of the transcription factors, thereby completing the present invention.

Furthermore, the phenome of *Cryptococcus neoformans* transcription factors according to the present invention can be easily accessed online (http://tf.cryptococcus.org), and provides a unique opportunity to understand general biological features of *C. neoformans,* and also provides novel targets required for the treatment of cryptococcosis.

US 2013/0237446 A1 describes uses of genes for HOG, Ras and cAMP signal transduction pathways to treat fungal infection.

### Prior Art Documents

### Patent Documents

(Patent Document 1) Korean Patent No. 10-1311196.
(Patent Document 2) Korean Patent No. 10-1403862.

### Non-Patent Documents

(Non-Patent Document 1) Park, B.J., and et al., 2009, AIDS, 23: 525-530.
(Non-Patent Document 2) Perfect, J.R., et al., 2010, Clinical infectious diseases, 2010 update by the infectious diseases society of America, 50: 291-322.
(Non-Patent Document 3) Bahn, Y.S., and Jung, K.W., 2013, Eukaryot. Cell. 12: 1564-1577.
(Non-Patent Document 4) Jung. W.H. and Kronstad. J.W., 2008, Cell. Microbiol., 10: 277-284.
(Non-Patent Document 5) Ding, C. et al., 2013, Cell Host Microbe, 13: 265-276.
(Non-Patent Document 6) Janbon, G., and et al., 2014, PLoS Genet. 10: el004261.
(Non-Patent Document 7) Kummerfeld, S.K., and Teichmann, S. A., 2006, Nucleic Acids Res., 34: D74-81.
(Non-Patent Document 8) Wilson, D., and et al., 2008, Nucleic Acids Res. 36: D88-D92.
(Non-Patent Document 9) Cheon. S.A., and et al., 2011, PLoS Pathog. 7: el002177.
(Non-Patent Document 10) Kim. M.S., and et al., 2010, Genetics.185: 1207-1219.
(Non-Patent Document 11) Song, M.H., and et al., 2012, Eukaryot. Cell. 11: 53-67.
(Non-Patent Document 12) Liu. O.W. and et al., 2008, Cell. 135: 174-188.
(Non-Patent Document 13) Homann, O.R., and et al., 2009, PLoS Genet. 5: e 1000783.
(Non-Patent Document 14) Inglis. D.O., and et al., 2014 , Eukaryot. Cell. 13: 878-883.
(Non-Patent Document 15) Davidson, R.C., and et al., 2002, Microbiology, 148: 2607-2615.
(Non-Patent Document 16) Kim. M.S., and et al., 2009, Biochem. Biophy. Res. Commun. 390: 983-988.
(Non-Patent Document 17) Lin, X., and Heitman, J., 2006, Annu. Rev. Microbiol. 60: 69-105.
(Non-Patent Document 18) Garcia-Rodas, R., and Zaragoza. O., 2012, Med Microbiol. 64: 147-161.
(Non-Patent Document 19) McFadden, D. C., and Casadevall, A. 2001, Med. Mycol. 39 Suppl 1:19-30.
(Non-Patent Document 20) Bahn, Y.S. and et al., 2004, Eukaryotic. Cell. 3: 1476-1491.
(Non-Patent Document 21) Mylonakis, E. et. al., 2005, Infect. Imraun. 73: 3842-3850.
(Non-Patent Document 22) Haynes, B.C., and et al., 2011, PLoS Pathog. 7: el002411.
(Non-Patent Document 23) Wickes, B.L., and et al., 1997, Mol. Microbiol. 26: 951-960.
(Non-Patent Document 24) Bahn, Y.S., and et al., 2006, Mol. Biol. Cell, 17: 3122-3135.
(Non-Patent Document 25) Jiang, N., and et al., 2009, FEMS Microbiol. Lett. 296: 84-90.
(Non-Patent Document 26) Pukkila-Worley, R., and et al., 2005, Eukaryot. Cell 4: 190-201.
(Non-Patent Document 27) Chang, Y.C., and et al., 2007, Mol. Microbiol., 64: 614-629.
(Non-Patent Document 28) Chun, C.D., and et al., 2007, PLoS Pathog., 3: e22.
(Non-Patent Document 29) Cramer, K.L., and et al., 2006, Eukaryot. Cell, 5: 1147-1156.
(Non-Patent Document 30) Idnurm, A., and Heitman, J.2005, PLoS Biol. 3: e95.
(Non-Patent Document 31) Alder, A., and et al., 2011, J. Biol. Chem., 286: 20977-20990.
(Non-Patent Document 32) Chun. C. D., and et al., 2011, Cell host & microbe, 9: 243-251.
(Non-Patent Document 33) Jung, W., and et al., 2011, Fungal Genet. Biol. 48: 154-165.
(Non-Patent Document 34) Livak. K. J. and Schmittgen, T.D., 2001, Methods. 25: 402-408.
(Non-Patent Document 35) Cowen, L.E., 2008, Nat. Rev. Microbiol. 6: 187-198.
(Non-Patent Document 36) Vermes, A., and et al., 2000, J Antimicrob. Chemother. 46: 171-179.
(Non-Patent Document 37) Choi, J. and et al., 2013, Nucleic Acids Res. 41: D714-719.
(Non-Patent Document 38) Hunter, S. et al., 2012, Nucleic Acids Res. 40: D306-312.
(Non-Patent Document 39) Bendtsen, J.D. and et al., 2004, J. Mol. Biol. 340: 783-795.
(Non-Patent Document 40) Nakai, K. and Horton, P., 1999, Trends Biochem. Sci. 24: 34-36.
(Non-Patent Document 41) Emanuelsson, O., and et al., 2000, J. Mol. Biol., 300: 1005-1016.
(Non-Patent Document 42) Emanuelsson, O., and et al., 1999, Protein Sci., 8: 978-984.
(Non-Patent Document 43) Bendtsen, J.D., and et al., 2004, Protein Eng. Des. Sel. 17: 349-356.
(Non-Patent Document 44) Cokol, M., and et al., 2000, EMBO reports 1: 411-415.
(Non-Patent Document 45) Sonnhammer, E.L., and et al., 1998, Proc. Int. Conf. Intelli. Syst. Mol. Biol 6: 175-812.
(Non-Patent Document 46) Jung, K. et al., 2008, BMC Genomics, 9: 586.

### [Disclosure]

### [Technical Problem]

Therefore, it is an object of the present disclosure to construct transcription factor networks in a *Cryptococcus neoformans* strain and to provide various uses of the transcription factors.

It is an object of the present invention to use said transcription factors in a method for screening an agent for preventing or treating fungal infection caused by a *Cryptococcus neoformans* strain

Another object of the disclosure albeit not part of the invention is to provide a method for screening a candidate that may have a synergistic effect when administered in combination with a conventional antifungal agent or meningitis-treating agent.

Still another object of the present disclosure is to provide a pharmaceutical composition that exhibits an antifungal effect or a meningitis-treating effect by increasing or inhibiting the expression of a transcription factor that regulates the virulence of a *Cryptococcus neoformans* strain and a gene that encodes the transcription factor, wherein the gene is bzp4

Yet another object of the present disclosure albeit not part of the invention is to provide a pharmaceutical composition that exhibits an antifungal effect or a meningitis-treating effect by increasing or inhibiting the expression of a transcription factor that regulates the antifungal agent susceptibility of a *Cryptococcus neoformans* strain, a transcription factor that regulates the growth of the strain, a transcription factor that regulates the mating of the strain, a transcription factor that regulates the responses to various external stresses, and genes that encode the transcription factors.

### [Technical Solution]

To achieve the above objects, the present invention provides a *Cryptococcus neoformans* strain deposited under accession number KCCM51291. The present invention also provides a method for screening an antifungal agent comprising the steps as defined in claim 1.

Embodiments comprising a method for screening an antifungal agent, wherein the virulence regulatory gene is not bzp4, an antifungal agent for co-administration or an agent for treating meningitis are not encompassed by the wording of the claims but are considered as useful for understanding the invention. In one embodiment, the present invention provides a method for screening an antifungal agent, comprising the steps of: (a) bringing a sample to be analyzed into contact with a cell line (deposited under accession number KCCM51291) containing an antifungal agent-targeting gene; (b) measuring the expression of the antifungal agent-targeting gene in the cell line; and (c) determining that the sample is the antifungal agent, when the expression of the antifungal agent-targeting gene is measured to be down-regulated or up-regulated, wherein the antifungal agent-targeting gene is any one gene selected from the group consisting of an antifungal agent resistance regulatory gene, a growth regulatory gene, a mating regulatory gene, and a gene that regulates responses to external stress.

In the embodiment of the method for screening an antifungal agent, the antifungal agent resistance regulatory gene is a gene that regulates resistance to any one antifungal agent selected from the group consisting of azole-, polyene-, 5-flucytocin- and phenylpyrazole-based antifungal agents.

In the embodiment of the method for screening an antifungal agent, as the antifungal agent-targeting gene, a gene, which increases sensitivity to the azole-based antifungal agent when its expression is down-regulated, is any one gene selected from the group consisting of *bzp3, hlh3, bzp*/*hxl1, sre1, riw101, bap2, hlh1, yap4, pip2, miz1, mln1, hob6, mbf1, met32, fzc46, bap1, fzc14, fzc2, liv4, hsf2, zfc6, fzc45, fzc30, asg1, stel2, liv1, fzc22, fzc31, pan1, bzp2, sp1*/*crz1, bzp5, hlh2, sxi1 alpha, fzc34, fzc40, fzc38* and *fzcl7;* a gene that increases sensitivity to the polyene-based antifungal agent is any one selected from the group consisting of *hob1, mbs1, jjj1, ert1, ecm22, gat201, zap104, sp1*/*crz1, fzc6, bzp5, hlh1, pip2, hcm1, bzp2, usv101, hob4, ste12, hob5, grf1, hel2, fzc45, asg1, fzc22, hob6, pan1, liv4, cuf1, fzc49, fzc1, bwc2, fap1, fzc44, fzc8, fzc23, gat204, nrg1, pip201, hlh2, rim101, fzc38, hlh3, bzp3, mln1, met32, zfc2, fzc40, fzc31* and *rum1;* a gene that increases sensitivity to the 5-flucytocin-based antifungal agent is any one gene selected from the group consisting of *nrg1, zfc2, bap1, mbs1, fzc6, bap2, bzp3, jjj1, hlh1, pip2, apn2, fzc46, hap2, fzc51, bzp5, hcm1* and *fzc19;* and a gene that increases sensitivity to the phenylpyrazole-based antifungal agent is any one gene selected from the group consisting of *usv101, ada2, bap1, fzc6, hlh1, pip2, fzc46, hap2, bzp1*/*hxl1, fkh2, livl, bap2, bzp2, fzc21, hlh3, yrm101, bzp5, gln3, zfc8, ddt1, fzc22, hob6, rlm1, mln1, liv4, pan1, fzc35, yrm103, fzc3, asg1, fzc41, fzc43, fzc51, hap1, fzc38, met32* and *fzc32.*

In the embodiment of method for screening an antifungal agent, as the antifungal agent-targeting gene, a gene, which increases sensitivity to the azole-based antifungal agent when its expression is up-regulated, is any one gene selected from the group consisting of *hob1, hap2, skn7, nrg1, mbs1, ppr1, jjj1, hcm1, ada2, fzc9, gat7, ert1, fkh2, ecm22, ddt1, gat1, yrm103, cuf1* and *fzc51*; a gene that increases sensitivity to the polyene-based antifungal agent is any one selected from the group consisting of *sre1, bap1, fzc51, skn7, clr1, bzp5, atf1* and *fzc4;* a gene that increases sensitivity to the 5-flucytocin-based antifungal agent is any one gene selected from the group consisting of *hlh3, rim101, gat204, hob3, fzc50, znf2* and *rds2;* and a gene that increases sensitivity to the phenylpyrazole-based antifungal agent is any one gene selected from the group consisting of *nrg1, jjj1, sp1*/*crz1, skn7, gat7, fap1, zfc2, gat204, znf2, hel2, fzc50* and *sre1.*

In the embodiment of the method for screening an antifungal agent, as the antifungal agent-targeting gene whose expression is down-regulated, a growth regulatory gene is temperature-independent or temperature-dependent. The temperature-independent growth regulatory gene is any one gene selected from the group consisting of *bzp2, cuf1, hob1, gat5, fzc6* and *nrg1;* and the temperature-dependent growth regulatory gene that regulates growth at a temperature of 37°C to 39°C is any one gene selected from the group consisting of *hxl1, crz1, atf1, ada2, liv4, aro80, usv101, fzc31, fzc30, mln1, fzc30, fzc1, miz1, apn2, gat6, mbs2, sre1* and *ert1.* Furthermore, as the antifungal agent-targeting gene whose expression is up-regulated, an antifungal agent-targeting gene that regulates growth at 39°C is any one gene of *mln1* and *fzc46.*

In the embodiment of the method for screening an antifungal agent, as the antifungal agent-targeting gene whose expression is down-regulated, a mating regulatory gene is any one gene selected from the group consisting of *bzp2, usv101, fzc1, zap104* and *skn7;* and as the antifungal agent-targeting gene whose expression is up-regulated, a mating regulatory gene is any one gene selected from the group consisting of *hlh1, hap2, skn7* and *gat1.*

In the embodiment of the method for screening an antifungal agent, the antifungal agent-targeting gene, which regulates responses to external stress when its expression is down-regulated, may be an antifungal agent-targeting gene that regulates responses to an osmotic stress induced by any one selected from the group consisting of 1M to 1.5M sodium chloride (NaCl), 1M to 1.5M potassium chloride (KCl) and 2M sorbitol. Specifically, the antifungal agent-targeting gene that regulates responses to the osmotic stress induced by the sodium chloride is any one gene selected from the group consisting of *rim101, skn7, ada2, fzc42, hcm1, gat7, bzp2, hob1, hap2, hob6, aro8001, pan1, fzc34, bap1, fzcl9, fzc51, fzc43, fzc13, gat5* and *met32;* the antifungal agent-targeting gene that regulates responses to the osmotic stress induced by the potassium chloride is any one gene selected from the group consisting of *bzp2, hob2, nrg1, hap2, ada2, fzc6, yrm103, fzc44, fzc32, hob1, rim101, bzp4* and *fzc35;* and the antifungal agent-targeting gene that regulates responses to the osmotic stress induced by the sorbitol is any one gene selected from the group consisting of *bzp2, hob1* and *fzc6.* Moreover, among antifungal agent-targeting genes that regulates responses to an oxidative stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 mM menadione, and 2 mM to 3 mM diamide (DA), the antifungal agent-targeting gene that regulates responses to the oxidative stress induced by the hydrogen peroxide is any one gene selected from the group consisting of *bap1, sre1, usv101, fzc50, fzc31, hob1, ada2, cuf1, gat204* , *ste12, fzc9, gat1, fzc21, nrg1, bzp2, gat5, pan1, met32, fzc4, rim101, hob6, fzcl3, hlh1, fzc46, sip402, fzc27, hob5, hob4, sp1(crz1), fzc22, bzp3, liv1, miz1* and *gat201;* the antifungal agent-targeting gene that regulates responses to the oxidative stress induced by the tert-butyl hydroperoxide is any one gene selected from the group consisting of *sre1, ada2, rim101, bap2, bap1, usv101, fzc31, hob1, fzc34, ecm22, fzc15, fzc44, zfc4, fzc49, yrm103, fzc21, zfc2, gat5, pan1, met32, hob4, liv1, mwc2, skn7, hcm1, fzc51, fzc1, ppr1, atf1, grf1, bzp5, gat8, clr1, hlh2, rlm1, fzc6, asg1, hob2,* and *zap103;* the antifungal agent-targeting gene that regulates responses to the oxidative stress induced by the menadione is any one gene selected from the group consisting of *bap1, fzc37, usv101, nrg1, bzp2, fzc4, fzc34, fzc35, hel2, ecm22, fzc6, gat6, jjj1, fzc44, fzc3* and *fzc26;* and the antifungal agent-targeting gene that regulates responses to the oxidative stress induced by the diamide is any one gene selected from the group consisting of *bap1, hob1, bap2, hap2, pip2, bzp5, hsf2, sre1, fzc21, zfc2, fzc31, bzp2, gat5, pan1, met32, fzc4, fzc34, hob6, hlh1, fzc46, sip402, fzc27, miz1, fzcl9, hlh3, fkh2, mln1, gat6, fap1, fzc8, fzc49, fzc3, fzc30, rum1* and *fzc38.* In addition, among antifungal agent-targeting genes that regulates responses to endoplasmic reticulum (ER) stress induced by 0.3 µg/ml tunicamycin (TM) or 20 mM dithiothreitol (DTT), the antifungal agent-targeting gene that regulates responses to the endoplasmic reticulum stress induced by the tunicamycin is any one gene selected from the group consisting of *bzp1 (hxl1), sre1, hlh1, bzp3, pip2, rlm1, met32, stel2, rim101, sp1 (crz1), fzc21, gat7, mln1, fzc2, fzc44, liv4, fzc40* and *fzc38;* and the antifungal agent-targeting gene that regulates responses to the endoplasmic reticulum stress induced by the dithiothreitol is any one gene selected from the group consisting of *bzp1 (hxl1), sre1, bzp2, cuf1, hob1 clr1, ada2, rlm1, gat5, hap2, nrg1, usv101, fzc31, gat201, hlh2, apn2, fzc25* and *ddt1.* In addition, among antifungal agent-targeting genes that regulates responses to a genotoxic stress induced by 0.03% to 0.06% methyl methanesulfonate (MM) or 50 mM to 100 mM hydroxyurea (HU), the antifungal agent-targeting gene that regulates responses to the genotoxic stress induced by the methyl methanesulfonate is any one gene selected from the group consisting of *bzp1 (hxl1), fzc6, hob1, sre1, gat5, gat6, miz1, bzp2, jjj1, fzc40, fzc38, fzc4, hcm101, fzc1* and *apn2;* and the antifungal agent-targeting gene that regulates responses to the genotoxic stress induced by the hydroxyurea is any one gene selected from the group consisting of *hob1, sre1, gat5, gat6, mbs1, skn7, ada2, bzp1 (hxl1), fzc6, bzp2, jjj1, hcm1, nrg1* and *hlh2.* In addition, among antifungal agent-targeting genes that regulates responses to a cell wall or cell membrane stress induced by any one selected from the group consisting of 3 mg/ml to 5 mg/ml calcofluor white (CFW), 0.8% to 1% Congo red (CR), and 0.03% sodium dodecyl sulfate (SDS), the antifungal agent-targeting gene that regulates responses to the cell wall or cell membrane stress induced by the CFW is any one gene selected from the group consisting of *bzp1 (hxl1), sp1 (crz1), hob1, hap2, bzp2, nrg1, bap2, rim101* and *pip2;* the antifungal agent-targeting gene that regulates responses to the cell wall or cell membrane stress induced by the CR is any one gene selected from the group consisting of *sp1 (crz1), hob1, bzp1 (hxl1), cuf1, hlh3, hap2, bzp2, nrg1, bap2* and *rim101;* and the antifungal agent-targeting gene that regulates responses to the cell wall or cell membrane stress induced by the SDS is any one gene selected from the group consisting of *sp1 (crz1), hob1, sre1, pip2, fzc21, gat7, hob3, usv101, gat201, fzc7, asg1, rum1, hap2, bzp2, nrg1, cuf1, gat5, gat6, jjj1, pan1, bzp3, rlm1, bap1, clr1, zfc4, clr4, gat1, fzc31, hob5, asg101, ert1, ecm22, zfc6, bzp5, sxi1 alpha, fap1, sip4, rds2, fzc26* and *fzc30.* In addition, an antifungal agent-targeting gene that regulates responses to a heavy-metal stress induced by 20 M to 30 M cadmium sulfate (CdSO₄) is any one gene selected from the group consisting of *cuf1, hap2, fzc6, skn7, fzc37, bzp2, gat5, yox101, mln1, pip2, hcm1, hob6, fzc46, hob5, mbs2, fzc35, aro8001, fzc19, fzc51, aro80, ccd4, fzc47, bzp4, fap1, fzc8, pip201, gln3, yrm101, zfc8, hob7, rum1* and *fzc10.*

In the embodiment of the method for screening an antifungal agent, the antifungal agent-targeting gene, which regulates responses to external stress when their expression is up-regulated, may be an antifungal agent-targeting genes that regulates an osmotic stress induced by any one selected from the group consisting of 1 M to 1.5 M sodium chloride (NaCl), 1 M to 1.5 M potassium chloride (KCl) and 2 M sorbitol. Specifically, the antifungal agent-targeting gene that regulates responses to the osmotic stress induced by the sodium chloride is any one gene selected from the group consisting of *hlh3, hel2* and *cuf1*; the antifungal agent-targeting gene that regulates responses to the osmotic stress induced by the potassium chloride is any one gene of *fzc36* and *yrm103*; and the antifungal agent-targeting gene that regulates responses to the osmotic stress induced by the sorbitol is *fzc36.* In addition, among antifungal agent-targeting genes that regulate responses to an oxidative stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 Mm menadione, and 2 mM to 3 mM diamide (DA), the antifungal agent-targeting gene that regulates responses to the oxidative stress induced by the hydrogen peroxide is any one gene selected from the group consisting of *fzc45, asg101, mbs2, fzc35, bwc2, fzc7* and *znf2;* the antifungal agent-targeting gene that regulates responses to the oxidative stress induced by the tert-butyl hydroperoxide is any one gene selected from the group consisting of *fzc33, fap1, clr3* and *ddt1;* the antifungal agent-targeting gene that regulates responses to the oxidative stress induced by the menadione is any one gene selected from the group consisting of *zfc2, fzc50, cuf1, hap2* and *sip4;* and the antifungal agent-targeting gene that regulates responses to the oxidative stress induced by the diamide is any one gene selected from the group consisting of *fzc50, sip4, pip201, nrg1, gat1, znf2, asg101, skn7, gat7, jjj1, hlh5, fzc26* and *fzc20.* In addition, among antifungal agent-targeting genes that regulate responses to an endoplasmic reticulum stress induced by 0.3 µg/ml tunicamycin (TM) or 20 mM dithiothreitol (DTT), the antifungal agent-targeting gene that regulates responses to the endoplasmic reticulum stress induced by the tunicamycin is any one gene selected from the group consisting of *bzp2, nrg1, hap2, cuf1, mbs1, ppr1, fzc6, skn7, zfc2, hob1, gat5, clr1, bap1, bwc2, hcm1, he12, gat6, jjj1, hob3, zfc4, zfc3* and *clr4*; and the antifungal agent-targeting gene that regulates responses to the endoplasmic reticulum stress induced by the dithiothreitol is any one gene selected from the group consisting of *yap4, hlh1, bzp3, pip2, pan1, mbs1, met32, gat1, fkh2, fzcl1, gat203, sip401, stb4* and *fzc20.* In addition, among antifungal agent-targeting genes that regulate responses to a genotoxic stress induced by 0.03% to 0.06% methyl methanesulfonate (MM) or 50 mM to 100 mM hydroxyurea (HU), the antifungal agent-targeting gene that regulates responses to the genotoxic stress induced by the methyl methanesulfonate is *yox1;* and the antifungal agent-targeting gene that regulates responses to the genotoxic stress induced by the hydroxyurea is *fzc20.* In addition, among antifungal agent-targeting genes that regulate responses to a cell wall or cell membrane stress induced by any one selected from the group consisting of 3 mg/ml to 5 mg/ml calcofluor white (CFW), 0.8 % to 1% Congo red (CR), and 0.03% sodium dodecyl sulfate (SDS), the antifungal agent-targeting gene that regulates responses to the cell wall or cell membrane stress induced by the CFW is any one gene of *fzc9* and *grf1*; and the antifungal agent-targeting gene that regulates responses to the cell wall or cell membrane stress induced by the SDS is any one gene selected from the group consisting of *fzc6, fzc1, zfc1, hsf3, bwc2, skn7, fzc50, fzc22, fzc51* and *fzc8.* In addition, an antifungal agent-targeting gene that regulates responses to a heavy-metal stress induced by 20 µM to 30 µM cadmium sulfate (CdSO₄) is any one gene selected from the group consisting of *bzp1 (hxl1), gat201, znf2, sip4, rds2, sre1, gat7, rlm1, clr1, zfc3, ada2, gat204, fzc7, asg101, atf1, hlh2, fzc39* and *hsf3.*

In another embodiment, the present disclosure provides a method for screening an antifungal agent, an antifungal agent for co-administration or an agent for treating meningitis, the method comprising the steps of: (a) bringing a sample to be analyzed into contact with a cell comprising a virulence regulatory gene; (b) measuring the expression of the virulence regulatory gene in the cell; and (c) determining that the sample is an antifungal agent, when the expression of the virulence regulatory gene is measured to be down-regulated or up-regulated.

In the embodiment of the method for screening an antifungal agent, an antifungal agent for co-administration or an agent for treating meningitis, the virulence regulatory gene may be a gene that regulates *Cryptococcus neoformans* pathogenicity. Specifically, the gene whose expression is down-regulated is any one gene selected from the group consisting of *usv101, fzc1, bap1, hob1, zfc2, fzc50, fzc31, bzp2, fzc9, ddt1, ma113, fzc2, fzc43, fzc22, hih1, mbs2, rum1, fzc5, aro80, clr1, pip2, fzc37, gat5, fzc49, cef3, fzc33, fzc12* and *zfc5*; and the gene whose expression is up-regulated is any one selected from the group consisting of *fzc17, fzc40, aro8001, fzc38, fzc24* and *ert1.*

In the embodiment of the method for screening an antifungal agent, an antifungal agent for co-administration or an agent for treating meningitis, the virulence regulatory gene regulates the production of any one selected from the group consisting of capsule, melanin and urease.

In the embodiment of the method for screening an antifungal agent, an antifungal agent for co-administration or an agent for treating meningitis, the gene that reduces capsule production is any one gene selected from the group consisting of *bap1, rds2, zapl04, fzc47, gat204, fzc33, fzc45, hsf2, bzp4, hob5, fzc16, hob3, zfc4, mcm1, liv4, hob4* and *liv1*; and the gene that increases capsule production is any one gene selected from the group consisting of *hob7, clr3, fzc51, fzc1, fkh2, nrg1, usv101, fzc29, bzp3, zfc3, fzc14, sre1, fzc30, hlh4, fzc36, crl6, mln1, fzc46, clr1, fzcl7, jjj1, fzc49, fzcl8, hcm1, fzc24, hlh3* and *hpa1.*

In the embodiment of the method for screening an antifungal agent, an antifungal agent for co-administration or an agent for treating meningitis, the gene that reduces melanin production is any one gene selected from the group consisting of *bzp4, fzc8, hob1, usv101, liv1, mbs2, fzc5, fzc25* and *ert1;* and the gene that increases melanin production is any one gene selected from the group consisting of *bzp2, fkh2, bapl, bzp3, hlh1, sip4, rds2, sip401, fzc1, gatl, ada2, nrg1, fzc31* and *hlh2.*

In the embodiment of the method for screening an antifungal agent, an antifungal agent for co-administration or an agent for treating meningitis, the gene that reduces urease production is any one gene selected from the group consisting of *zap104, sre1, gat201, fzc46, hlh1* and *fzc21*; and the gene that increases urease production is any one gene selected from the group consisting of *rim1, atf1, fkh2, fzc1, usv101, bap1, sxi1 alpha, mln1, fzc26, skn7, zfc7, hob7, fzc14* and *hob4.*

In the embodiment of the method for screening an antifungal agent, an antifungal agent for co-administration or an agent for treating meningitis, the cell is *Cryptococcus neoformans.*

The term "sample" as used herein with reference to the screening method means an unknown candidate that is used in screening to examine whether it influences the expression of a gene or the amount or activity of a protein. Examples of the sample include, but are not limited to, chemical substances, nucleotides, antisense-RNA, siRNA (small interference RNA) and natural extracts.

The term "antifungal agent" as used herein is meant to include inorganic antifungal agents, organic natural extract-based antifungal agents, organic aliphatic compound-based antifungal agents, and organic aromatic compound-based antifungal agents, which serve to inhibit the propagation of bacteria and/or fungi. Examples of the inorganic antifungal agents include, but are not limited to, chlorine compounds (especially sodium hypochlorite), peroxides (especially hydrogen peroxide), boric acid compounds (especially boric acid and sodium borate), copper compounds (especially copper sulfate), zinc compounds (especially zinc sulfate and zinc chloride), sulfur-based compounds (especially sulfur, calcium sulfate, and hydrated sulfur), calcium compounds (especially calcium oxide), silver compounds (especially thiosulfite silver complexes, and silver nitrate), iodine, sodium silicon fluoride, and the like. Examples of the organic natural extract-based antifungal agents include hinokithiol, *Phyllostachys pubescens* extracts, creosote oil, and the like.

The term "meningitis" as used herein is meant to include various inflammatory diseases occurring in the subarachnoid space between the arachnoid and the pia mater, for example, those caused by invasion of viruses or bacteria into the subarachnoid space, inflammation caused by a certain chemical substance, and those caused by the spread of cancer cells into the cerebrospinal fluid space.

### [Advantageous Effects]

The present disclosure may effectively screen a composition having an antifungal effect or a meningitis-treating effect by measuring the expression level of a transcription factor that regulates virulence in a *Cryptococcus neoformans* strain. In addition, the present disclosure may provide a pharmaceutical composition, which exhibits an antifungal effect or a meningitis-treating effect, by up-regulating or down-regulating the expression of a transcription factor that regulates *Cryptococcus neoformans* virulence.

### [Description of Drawings]

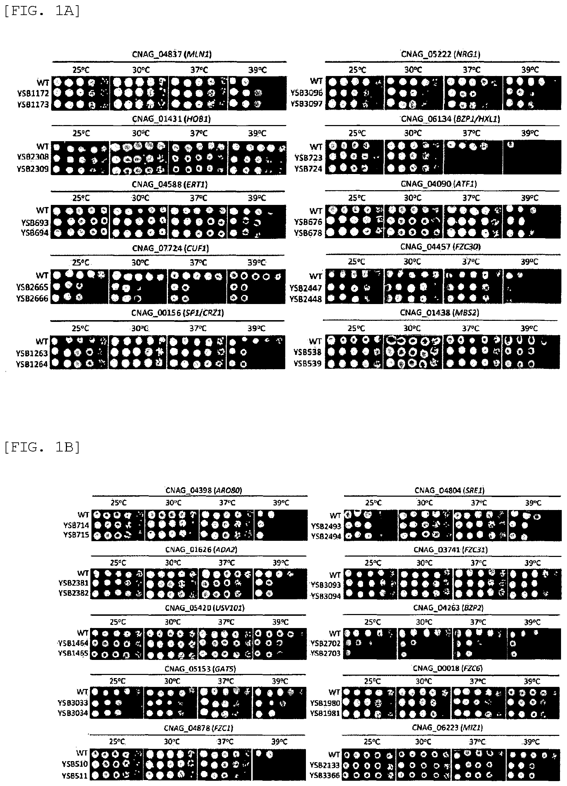

FIGS. 1a and 1b show transcription factors required for the temperature-dependent growth of *Cryptococcus neoformans.*

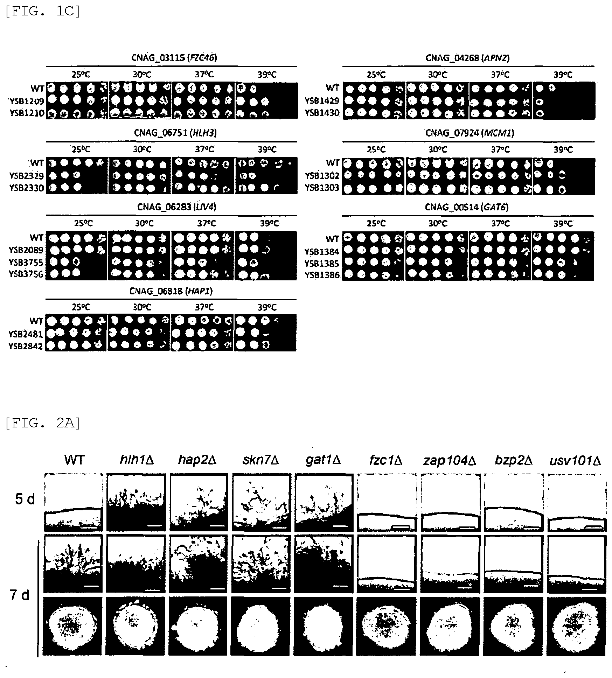

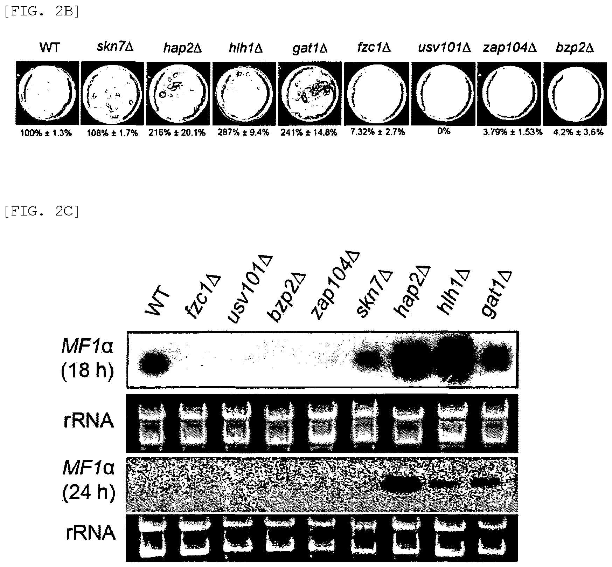

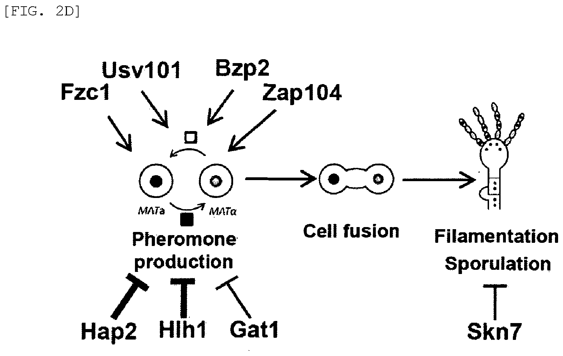

FIGS. 2a to 2d show transcription factors involved in sexual differentiation of *Cryptococcus neoformans.* Specifically, FIG. 2a shows the results of a mating assay in which the WT strain H99 and each TF mutant were cocultured with the opposite mating type KN99a strain on V8 media and incubated at room temperature in the dark for 7 days; FIG. 2b shows the cell-fusion efficiency of each TF mutant calculated relative to that of control strains (NAT-marked wild-type strain (YSB119) × NEO-marked wild-type a strain (YSB121)); FIG. 2c shows transcription factors involved in pheromone gene expression. In FIG. 2c, indicated transcription factor mutants were cocultured with the KN99a strain on V8 medium at room temperature for 18 to 24hours, and then RNA expression was analyzed. FIG. 2d is a schematic diagram showing the role of transcription factors in various mating stages of *Cryptococcus neoformans.*

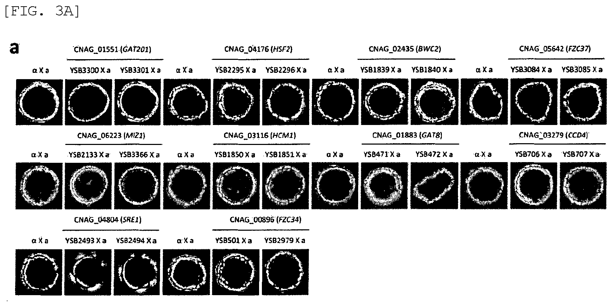

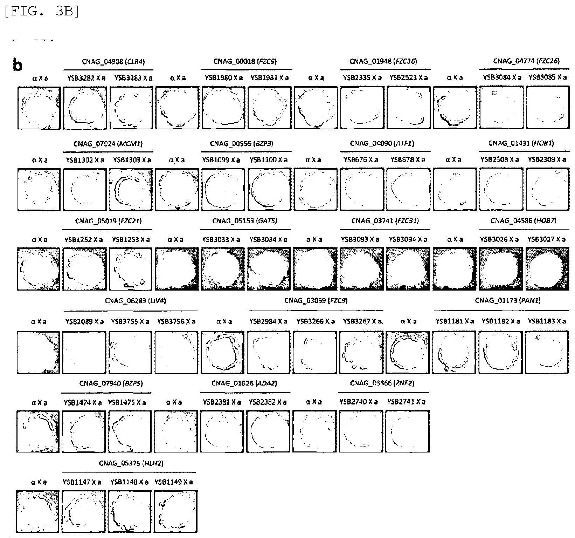

FIGS. 3a and 3b show transcription factors involved in mating efficiency (sexual differentiation) in *Cryptococcus neoformans* (3a-positive regulators; 3b-negative regulators).

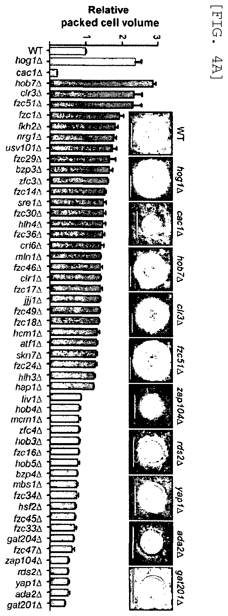

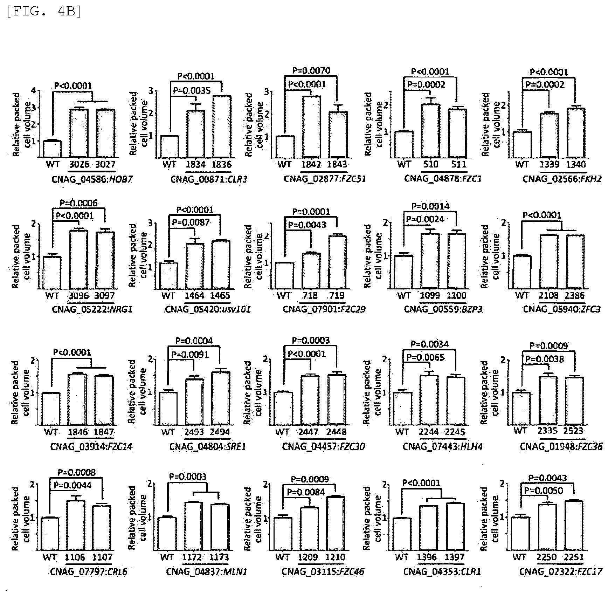

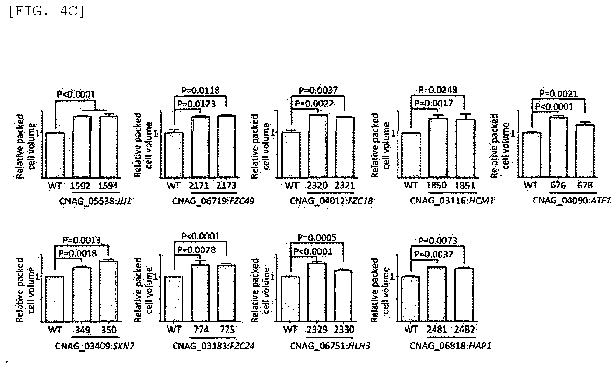

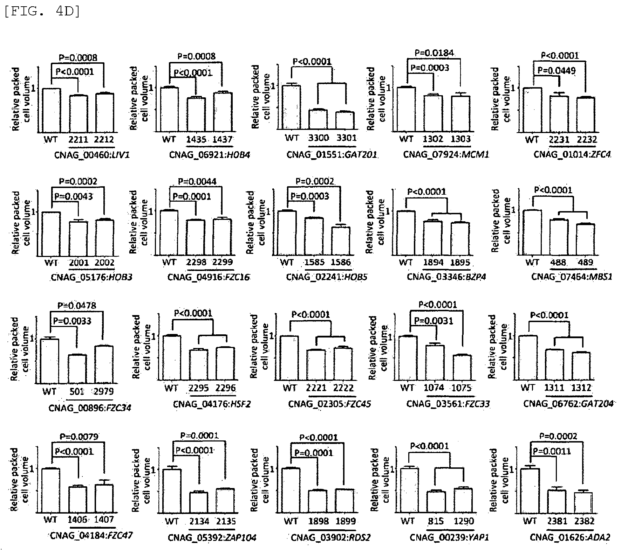

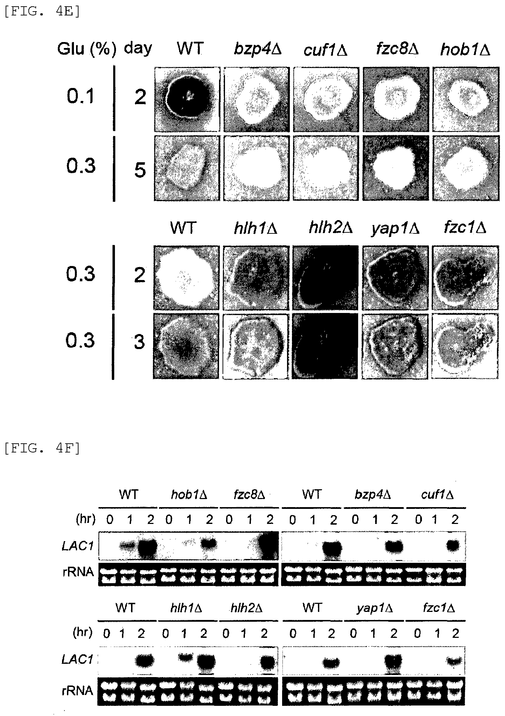

FIGS. 4a to 4f show transcription factors involved in virulence-factor production in *Cryptococcus neoformans.* Specifically, FIG. 4a shows the sizes of capsules produced in the wild-type strain H99 and transcription factor mutants; FIGS. 4b and 4c show transcription factors involved in capsule production in *Cryptococcus neoformans* (4b-negative regulators; 4c-positive regulators); and FIGS. 4d and 4e show the correlation between the expression of LAC1, which is the major laccase involved in melanin synthesis, and transcription factors. Specifically, FIG. 4d shows the results obtained by spotting transcription factor mutants on Niger seed agar medium (containing 0.1 and 0.3% glucose) and photographing the plates while culturing the mutants at 37°C; and FIG. 4e shows the results of Northern blot analyses performed using a LAC1-specific probe for total RNA isolated from cells under glucose-rich (0hr) and glucose-depleted conditions (1 and 2hr) .

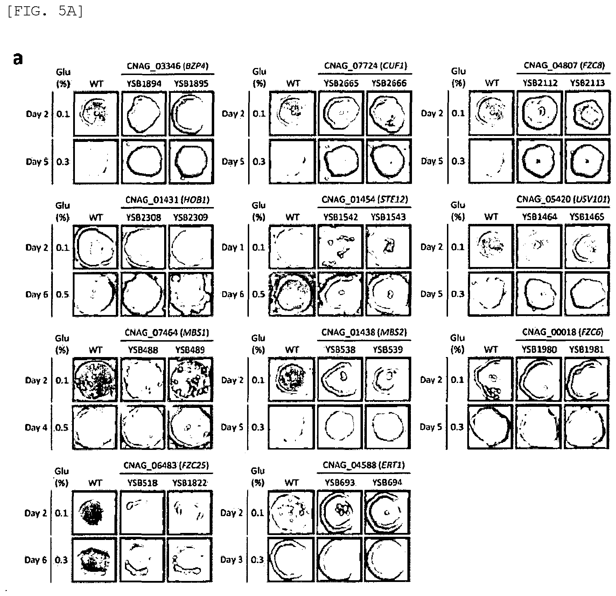

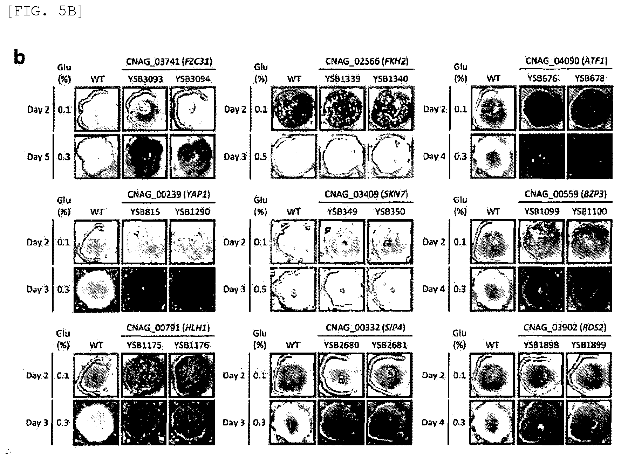

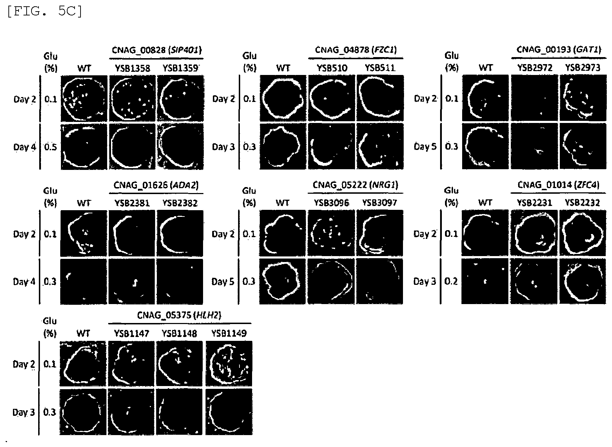

FIGS. 5a to 5c show transcription factors involved in melanin production in *Cryptococcus neoformans* (5a-positive regulators; 5b and 5c-negative regulators).

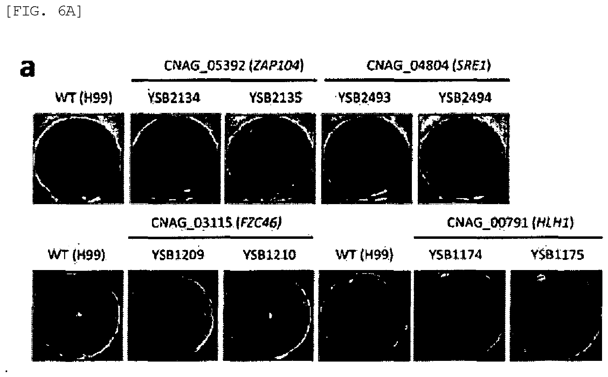

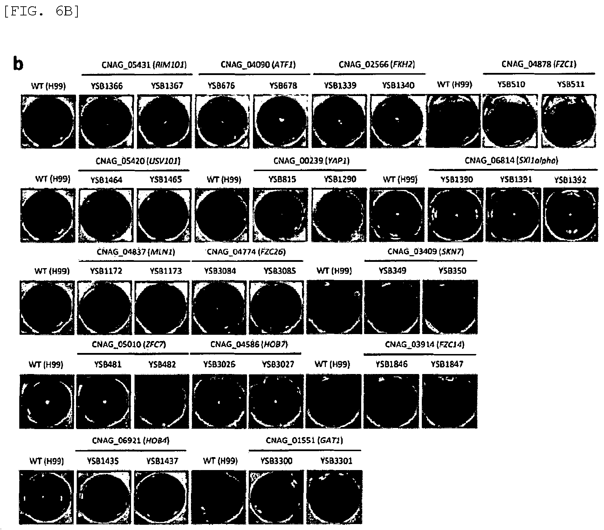

FIGS. 6a and 6b shows transcription factors required for urease production in *Cryptococcus neoformans* (6a-negative regulators; 6b-positive regulators).

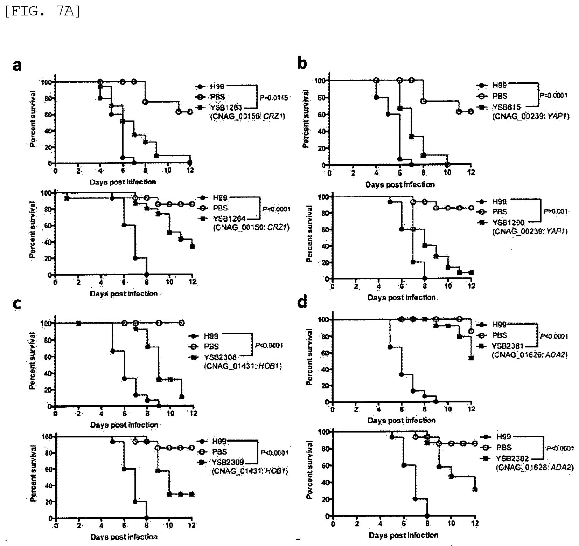

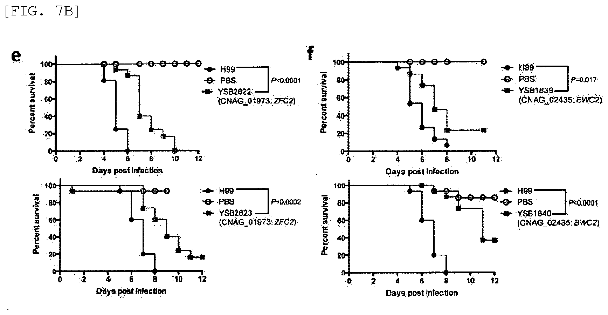

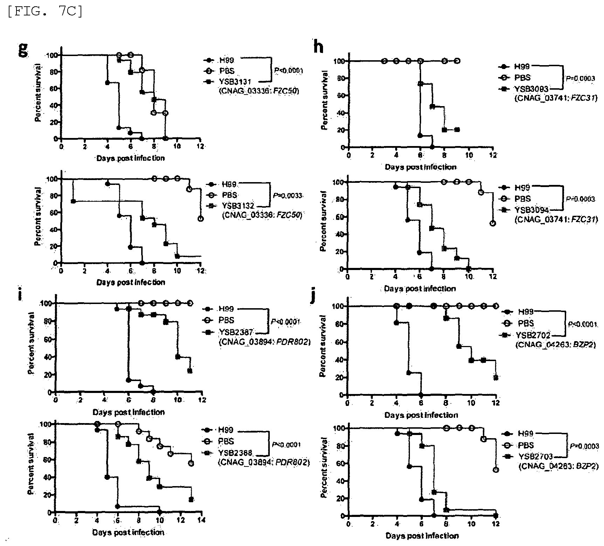

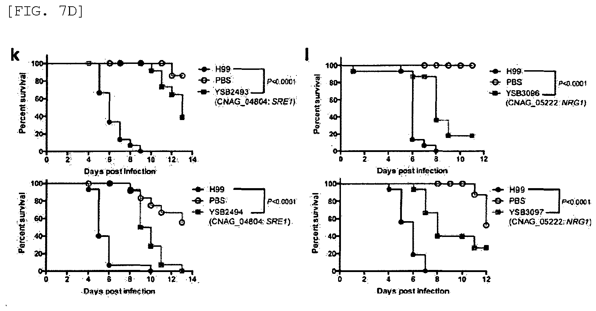

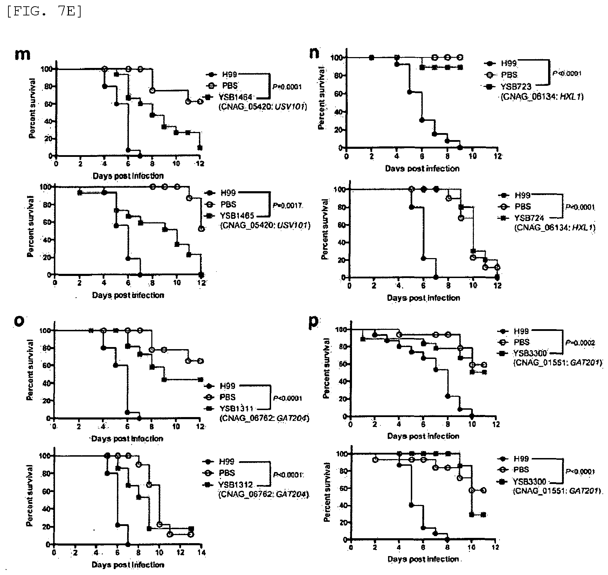

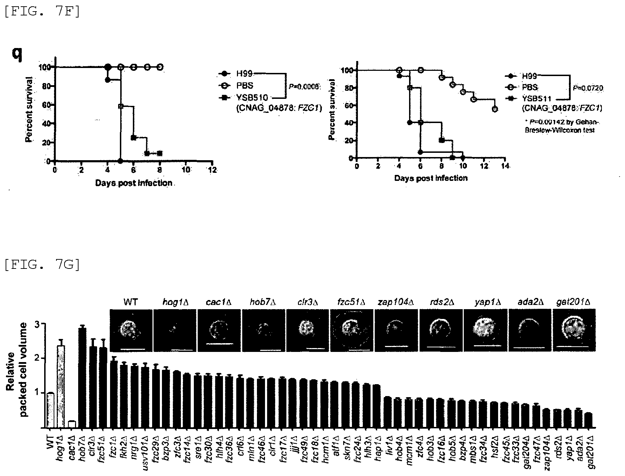

FIGS. 7a to 7g show transcription factors involved in *Cryptococcus neoformans* virulence in *Galleria mellonella* killing assay. Specifically, FIGS. 7a to 7f show the results obtained for various transcription factor deletions, and FIG. 7g shows the results of identifying virulence-related transcription factors in *Cryptococcus neoformans* by signature-tagged mutagenesis (STM)-based murine infectivity assay.

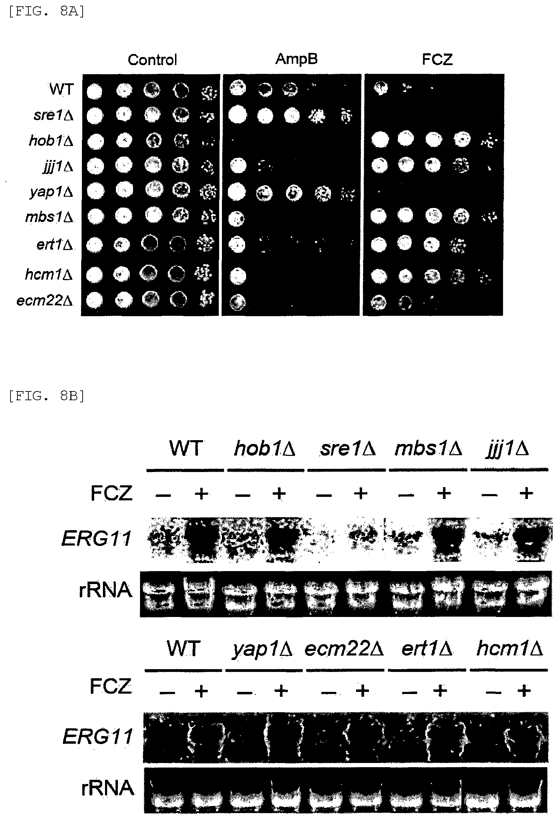

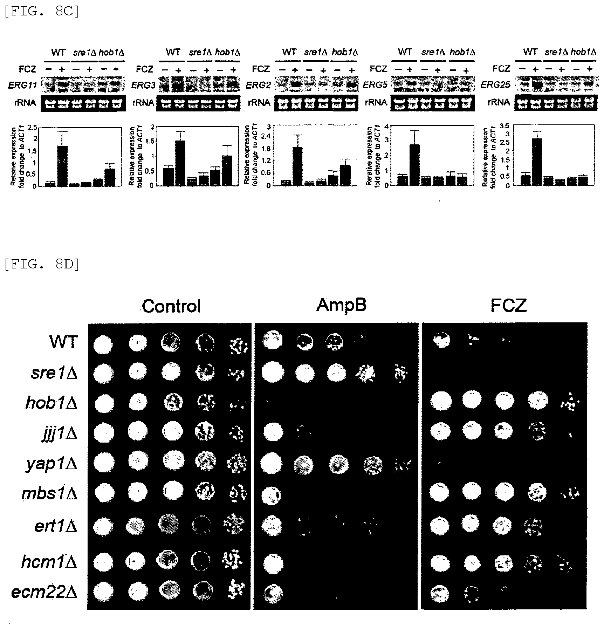

FIGS. 8a to 8d show that transcription factors regulating sterol biosynthesis genes govern general environmental stress responses and adaptation in *Cryptococcus neoformans.* FIG. 8a shows the results of observing the susceptibility of eight transcription factor mutants to antifungal drugs; FIG. 8b shows the results of Northern blot analysis performed using an ERG11-specific probe for the susceptibility of various transcription factor mutants to fluconazole (FCZ); FIG. 8c shows the results of Northern blot analysis performed using an ERG gene-specific probe for the susceptibility of a *sre1* mutant and *hob1* mutant to fluconazole (FCZ); FIG. 8d shows the results of observing the responses of a *sre1* mutant and *hob1* mutant to stress-inducing agents; and FIG. 8e shows a proposed model for the role of Sre1 and Hob1 in the sterol homeostasis and general stress responses of *Cryptococcus neoformans.*
FIGS. 9a and 9b show transcription factors involved in the virulence of *Cryptococcus neoformans.*

### [Mode for Invention]

Hereinafter, the present invention will be described in further detail with reference to examples. It will be obvious to those skilled in the art that these examples are for illustrative purposes and are not intended to limit the scope of the present invention as defined in the appended claims.

### Example 1: Construction of Transcription Factor Mutants

### 1.1: Selection of Transcription Factors and Transcription Factor Mutants

Putative transcription factors were screened using the published DBD transcription factor (TF) prediction database (http://www.transcriptionfactor.org/) (Non-Patent Document 8). The *Cryptococcus neoformans* H99 strain (a serotype A genome-sequence platform strain) contains 188 transcription factors (148 predicted from Pfam and 96 from SUPERFAMILY). Because these transcription factors were predicted based on the first version of the annotated H99 genome database, the present inventors updated this database with reference to the most recent version (version 7) of the annotated H99 genome database (Non-Patent Document 6), which resulted in a final prediction of 155 transcription factors (Table 1 below). The result of Orthologue mapping based on the BLAST e-value matrix demonstrated that *Cryptococcus neoformans* contains several evolutionarily distinct groups of transcription factors. The Cryptococcus DNA binding domain (DBD) transcription factors were classified based on their DNA binding domains (DBDs). 44% of these transcription factors (78) contain a fungal Zn2-Cys6 DBD, and among these, 40 also harbor a fungal-specific transcription factor domain. Several transcription factors contain more than two transcription factor domains (Table 1).

**[Table 1]**

| List of *Cryptococcus neoformans* transcription factors predicted based on DNA-binding domain database | | | | |
|---|---|---|---|---|
| No. | H 9 9 ID | TF domains | **Gene Name** | |
| 1 | 02566 | "Winged helix" DNA-binding domain / Fork head domain | | *FKH2* |
| 2 | 00791 | Helix-loop-helix DNA-binding domain | | *HLH1* |
| 3 | 01069 | Fungal Zn(2)-Cys(6) binuclear cluster domain / Fungal specific transcription factor domain | | *FZC11* |
| 4 | 07464 | APSES domain / Ankyr in repeat#2 | | *MBS1* |
| 5 | 03401 | Glucocorticoid receptor-like (DNA-binding domain) / GATA zinc finger | | *GAT203* |
| 6 | 04588 | Fungal Zn(2)-Cys(6) binuclear cluster domain | | *ERT1* |
| 7 | 00828 | Fungal Zn(2)-Cys(6) binuclear cluster domain / Fungal specific transcription factor domain | | *SIP401* |
| 8 | 03561 | Fungal Zn(2)-Cys(6) binuclear cluster domain / Fungal specific transcript ion factor domain | | *FZC33* |
| 9 | 06762 | Glucocorticoid receptor-like (DNA-binding domain) / GATA zinc finger | | *GAT204* |
| 10 | 06276 | Fungal Zn(2)-Cys(6) binuclear cluster domain / Fungal specific transcription factor domain | | *CEP3* |
| 11 | 05785 | Fungal Zn(2)-Cys(6) binuclear cluster domain | | *STB4* |
| 12 | 03132 | Fungal Zn(2)-Cys(6) binuclear cluster domain | | *FZC5* |
| 13 | 01438 | Ki IA-N domain / Ankyr in repeats (many copies) | | *MBS2* |
| 14 | 07593 | bZIP transcription factor / Domain of unknown function (DUF3425) | | *YAP4* |
| 15 | 04837 | Helix-loop-helix DNA-binding domain | | *MLN1* |
| 16 | 05093 | Homeobox domain | | *HOB6* |
| 17 | 05642 | Fungal Zn(2)-Cys(6) binuclear cluster domain / Fungal specific transcription factor domain | | *FZC37* |
| 18 | 05431 | Zinc-finger double domain / C2H2-type zinc finger | | *RIM101* |
| 19 | 04398 | Fungal specific transcription factor domain / Fungal Zn(2)-Cys(6) binuclear cluster domain | | *AR080* |
| 20 | 04878 | Fungal Zn(2)-Cys(6) binuclear cluster domain / Fungal specific transcription factor domain | | *FZC1* |
| 21 | 03183 | Fungal Zn(2)-Cys(6) binuclear cluster domain / Fungal specific transcription factor domain | | *FZC24* |
| 22 | 03710 | Fungal Zn(2)-Cys(6) binuclear cluster domain / Fungal specific transcription factor domain | | *ECM22* |
| 23 | 03279 | Fungal Zn(2)-Cys(6) binuclear cluster domain / Fungal specific transcription factor domain | | *CCD4* |
| 24 | 04637 | Helix-turn-helix/lambda repressor-like DNA-binding domains | | *MBF1* |
| 25 | 06425 | Fungal Zn(2)-Cys(6) binuclear cluster domain / Fungal specific transcription factor domain | | *PPR1* |
| 26 | 04345 | Fungal Zn(2)-Cys(6) binuclear cluster domain | | AR08001 |
| 27 | 04184 | Fungal Zn(2)-Cys(6) binuclear cluster domain / Fungal specific transcription factor domain | | *FZC47* |
| 28 | 02774 | Fungal Zn(2)-Cys(6) binuclear cluster domain / Fungal specific transcription factor domain | | *MAL13* |
| 29 | 00670 | Fungal Zn(2)-Cys(6) binuclear cluster domain | | *FZC12* |
| 30 | 00068 | Zinc-finger double domain / C2H2-type zinc finger | | *MET32* |
| 31 | 05010 | C2H2-type zinc finger | | *ZFC7* |
| 32 | 04090 | bZIP transcription factor / Basic region leucine zipper | | *ATF1* |
| 33 | 06134 | bZIP transcription factor / Basic region leucine zipper | | *BZP1(HXL1)* |
| 34 | 04630 | bZIP transcription factor / Basic region leucine zipper | | *BAP2* |
| 35 | 07901 | Fungal Zn(2)-Cys(6) binuclear cluster domain / Fungal specific transcription factor domain | | *FZC29* |
| 36 | 01173 | Beta-trefoil DNA-binding domain / p53-like transcription factors / DNA-binding protein LAG-1 (CSL) | | *PAN1* |
| 37 | 03115 | Fungal Zn(2)-Cys(6) binuclear cluster domain / Fungal specific transcription factor domain | | *FZC46* |
| 38 | 07924 | SRF-type transcript ion factor (DNA-binding and dimerisation | | *MCM1* |
| 39 | 07435 | CCAAT-binding transcription factor (CBF-B/NF-YA) subunit B | | *HAP2* |
| 40 | 02555 | Fungal Zn(2)-Cys(6) binuclear cluster domain / Fungal specific transcription factor domain | | *SIP402* |
| 41 | 04594 | Fungal Zn(2)-Cys(6) binuclear cluster domain / Fungal specific transcription factor domain | | *FZC27* |
| 42 | 06188 | Fungal Zn(2)-Cys(6) binuclear cluster domain | | *FZC15* |
| 43 | 05170 | Fungal Zn(2)-Cys(6) binuclear cluster domain | | *PIP2* |
| 44 | 02241 | Homeodomain-like / Helix-turn-helix domain | | *HOB5* |
| 45 | 06921 | Homeobox domain | | *H0B4* |
| 46 | 05186 | GRF zinc finger | | *GRF1* |
| 47 | 00896 | Fungal Zn(2)-Cys(6) binuclear cluster domain / Fungal specific transcription factor domain | | *FZC34* |
| 48 | 00039 | C2H2-type zinc finger | | *ZFC6* |
| 49 | 07940 | Basic region leucine zipper / bZIP transcription. factor | | *BZP5* |
| 50 | 06814 | Homeobox KN domain | | *SXI1alpha* |
| 51 | 01454 | STE like transcription factor / Zinc-finger double doma in / C2H2-type zinc finger | | *STE12* |
| 52 | 03527 | C2H2-type zinc finger / C3HC4-type zinc finger | | *HEL2* |
| 53 | 05255 | Fungal Zn(2)-Cys(6) binuclear cluster domain / Fungal specific transcription factor domain | | *FZC2* |
| 54 | 05112 | Fungal Zn(2)-Cys(6) binuclear cluster domain / Fungal specific transcription factor domain | | *FZC42* |
| 55 | 01883 | Glucocorticoid receptor-like (DNA-binding domain) / GATA zinc finger | | *GAT8* |
| 56 | 04353 | C2H2-type zinc finger | | *CLR1* |
| 57 | 05375 | Helix-loop-helix DNA-binding domain | | *HLH2* |
| 58 | 03998 | SRF-type transcription factor (DNA-binding and dimerisation | | *RLM1* |
| 59 | 00239 | bZIP transcription factor / Basic region leucine zipper | | *BAP1* |
| 60 | 06871 | Fungal Zn(2)-Cys(6) binuclear cluster domain / Fungal specific transcription factor domain | | *FZC41* |
| 61 | 00156 | C2H2-type zinc finger | | *SP1(CRZ1)* |
| 62 | 04268 | GRF zinc finger | | *APN2* |
| 63 | 05420 | Zinc-finger double domain / C2H2-type zinc finger | | *USV101* |
| 64 | 00018 | Fungal Zn(2)-Cys(6) binuclear cluster domain | | *FZC6* |
| 65 | 03346 | bZIP transcription factor | | *BZP4* |
| 66 | 00514 | Glucocort icoid receptor-like (DNA-binding domain) / GATA zinc finger | | *GAT6* |
| 67 | 05538 | SRR1 domain / C2H2-type zinc finger / DnaJ domain | | *JJJ1* |
| 68 | 03409 | "Winged helix" DNA-binding domain / CheY-like / HSF-type DNA-binding | | *SKN7* |
| 69 | 06339 | Fungal Zn(2)-Cys(6) binuclear cluster domain / Fungal specific transcription factor domain | | *FZC35* |
| 70 | 07011 | Fungal Zn(2)-Cys(6) binuclear cluster domain / Fungal specific transcription factor domain | | *FZC22* |
| 71 | 07506 | NF-X1 type zinc finger #3 / R3H domain | | *FAP1* |
| 72 | 04807 | Fungal Zn(2)-Cys(6) binuclear cluster domain | | *FZC8* |
| 73 | 02435 | PYP-like sensor domain (PAS domain) / Glucocorticoid receptor-like (DNA-binding domain) / GATA zinc finger / AT hook motif | | *B W C 2 (CWC2*) |
| 74 | 02364 | Fungal Zn(2)-Cys(6) binuclear cluster domain | | *FZC19* |
| 75 | 03116 | "Winged helix" DNA-binding domain / Fork head domain | | *HCM1* |
| 76 | 02877 | Zinc-finger double domain / Fungal Zn(2)-Cys(6) binuclear cluster domain | | *FZC51* |
| 77 | 00559 | bZIP transcription factor / Basic region leucine zipper | | *BZP3* |
| 78 | 03914 | Fungal Zn(2)-Cys(6) binuclear cluster domain / Fungal specific transcription factor domain | | *FZC14* |
| 79 | 00871 | bZIP transcription factor / Basic region leucine zipper | | *CLR3* |
| 80 | 06483 | Fungal Zn(2)-Cys(6) binuclear cluster domain | | *FZC25* |
| 81 | 07797 | Put at ive FMN-binding domain | | *CRL6* |
| 82 | 05019 | Fungal specific transcription factor domain | | *FZC21* |
| 83 | 05380 | Fungal Zn(2)-Cys(6) binuclear cluster domain / Fungal specific transcript ion factor domain | | *FZC44* |
| 84 | 04518 | Fungal specific transcription factor domain / C2H2-type zinc finger | | *ZFC5* |
| 85 | 05176 | Homeobox domain | | *HOB3* |
| 86 | 05861 | Fork head domain | | *FKH101* |
| 87 | 00460 | Helix-loop-helix DNA-binding domain | | *LIV1* |
| 88 | 02305 | Fungal Zn(2)-Cys(6) binuclear cluster domain / Fungal specific transcription factor domain | | *FZC45* |
| 89 | 03849 | Fungal specific transcription factor domain / Fungal Zn(2)-Cys(6) binuclear cluster domain | | *ASG1* |
| 90 | 01014 | C2H2-type zinc finger | | *ZFC4* |
| 91 | 01858 | Homeobox domain | | *HOB2* |
| 92 | 06719 | Fungal Zn(2)-Cys(6) binuclear cluster domain / Fungal specific transcription factor domain | | *FZC49* |
| 93 | 04093 | Fungal Zn(2)-Cys(6) binuclear cluster domain / Fungal specific transcription factor domain | | *YRM103* |
| 94 | 04176 | "Winged helix" DNA-binding domain / HSF-type DNA-binding | *HSF2* | |
| 95 | 01431 | Homeobox domain | *HOB1* | |
| 96 | 07443 | Helix-loop-helix DNA-binding domain | *HLH4* | |
| 97 | 04916 | Fungal Zn(2)-Cys(6) binuclear cluster domain | *FZC16* | |
| 98 | 05392 | Zinc-finger double domain / C2H2-type zinc finger | *ZAP104* | |
| 99 | 02322 | Fungal Zn(2)-Cys(6) binuclear cluster domain | *FZC17* | |
| 100 | 04012 | Fungal Zn(2)-Cys(6) binuclear cluster domain | *FZC18* | |
| 101 | 00505 | Fungal Zn(2)-Cys(6) binuclear cluster domain | *FZC28* | |
| 102 | 06751 | Helix-loop-helix DNA-binding domain | *HLH3* | |
| 103 | 04841 | Fungal Zn(2)-Cys(6) binuclear cluster domain / Fungal specific transcription factor domain | *FZC43* | |
| 104 | 03212 | "Winged helix" DNA-binding domain | *HCM101* | |
| 105 | 01626 | Zinc finger, ZZ type / Myb-like DNA-binding domain / SWIRM domain | *ADA2* | |
| 106 | 3894 | Fungal Zn(2)-Cys(6) binuclear cluster domain | *PDR802* | |
| 107 | 2066 | Fungal Zn(2)-Cys(6) binuclear cluster domain | *FZC13* | |
| 108 | 6818 | Fungal Zn(2)-Cys{6) binuclear cluster domain / Fungal specific transcription factor domain | *HAP1* | |
| 109 | 5940 | C2H2-type zinc finger | *ZFC3* | |
| 110 | 1948 | Fungal Zn(2)-Cys(6) binuclear cluster domain / Fungal specific transcription factor domain | *FZC36* | |
| 111 | 4804 | Helix-loop-helix DNA-binding domain | *SRE1* | |
| 112 | 4352 | Zinc-finger double domain / C2H2-type zinc finger | *ZAP103* | |
| 113 | 3768 | Fungal Zn(2)-Cys(6) binuclear cluster domain / Fungal specific transcription factor domain | *FZC32* | |
| 114 | 1977 | Fungal Zn(2)-Cys(6) binuclear cluster domain | *FZC39* | |
| 115 | 4895 | Fungal Zn(2)-Cys(6) binuclear cluster domain / Fungal specific transcription factor domain | *FZC3* | |
| 116 | 4583 | WSTF, HB1, Itc1p, MBD9 motif 2/3 / DDT domain | *DDT1* | |
| 117 | 1973 | Zinc-finger double domain / C2H2-type zinc finger / Fungal specific transcription factor domain | *ZFC2* | |
| 118 | 2603 | Fungal specific transcription factor domain / C2H2-type zinc finger | *ZFC1* | |
| 119 | 4036 | "Winged helix" DNA-binding domain / HSF-type DNA-binding | *HSF3* | |
| 120 | 6156 | Fungal Zn(2)-Cys(6) binuclear cluster domain / Fungal specific transcription factor domain | *FZC7* | |
| 121 | 03431 | Fungal Zn(2)-Cys(6) binuclear cluster domain / Fungal specific transcription factor domain | *FZC48* | |
| 122 | 07922 | Fungal Zn(2)-Cys(6) binuclear cluster domain / Fungal specific transcription factor domain | *FZC4* | |
| 123 | 00031 | Fungal Zn(2)-Cys(6) binuclear cluster domain / Fungal specific transcription factor domain | *MLR1* | |
| 124 | 03018 | Fungal Zn(2)-Cys(6) binuclear cluster domain/ Fungal specific transcription factor domain | *ASG101* | |
| 125 | 04263 | Glucocorticoid receptor-like (DNA-binding domain) / Basic region leucine zipper / bZIP transcript ion factor / GATA zinc finger | *BZP2* | |
| 126 | 01708 | GATA zinc finger | *GAT7* | |
| 127 | 00332 | Fungal Zn(2)-Cys(6) binuclear cluster domain | *SIP4* | |
| 128 | 07724 | Copper fist DNA binding domain | *CUF1* | |
| 129 | 03902 | Fungal Zn(2)-Cys(6) binuclear cluster domain/PAS fold | *RDS2* | |
| 130 | 03366 | Zinc-finger double domain / C2H2-type zinc finger | Z*NF2* | |
| 131 | 05222 | C2H2-type zinc finger / Zinc-finger double domain | *NRG1* | |
| 132 | 05049 | Fungal Zn(2)-Cys(6) binuclear cluster domain / Fungal specific transcription factor domain | *PIP201* | |
| 133 | 02516 | Helix-loop-helix DNA-binding domain | *HLH5* | |
| 134 | 04774 | Fungal Zn(2)-Cys(6) binuclear cluster domain / Fungal specific transcription factor domain | *FZC26* | |
| 135 | 03059 | Fungal Zn(2)-Cys(6) binuclear cluster domain | *FZC9* | |
| 136 | 00193 | Glucocort icoid receptor-like (DNA-binding domain) / GATA zinc finger | *GAT1* | |
| 137 | 03336 | Fungal Zn(2)-Cys(6) binuclear cluster domain / Fungal specific transcription factor domain | *FZC50* | |
| 138 | 03086 | Fungal Zn(2)-Cys(6) binuclear cluster domain / Fungal specific transcription factor domain | *FZC20* | |
| 139 | 03229 | Homeobox domain / Homeobox KN domain | *YOX101* | |
| 140 | 01841 | Glucocorticoid receptor-like (DNA-binding domain) / GATA zinc finger | *GLN3* | |
| 141 | 02476 | Fungal Zn(2)-Cys(6) binuclear cluster domain / Fungal specific transcription factor domain | *YRM101* | |
| 142 | 05153 | Glucocorticoid receptor-like (DNA-binding domain) / GATA zinc finger / AT hook motif | *GAT5* | |
| 143 | 02700 | C2H2-type zinc finger | *ZFC8* | |
| 144 | 04586 | Homeobox domain | *HOB7* | |
| 145 | 2723 | Fungal Zn(2)-Cys(6) binuclear cluster domain / Fungal specific transcription factor domain | *FZC23* | |
| 146 | 3741 | Fungal Zn(2)-Cys(6) binuclear cluster domain / AT hook motif | *FZC31* | |
| 147 | 4457 | Fungal Zn(2)-Cys(6) binuclear cluster domain / AT hook motif | *FZC30* | |
| 148 | 6283 | Homeodomain-like | *LIV4* | |
| 149 | 7411 | C5HC2 zinc finger / PHD-finger / ARID/BRIGHT DNA binding | *RUM1* | |
| 150 | 4836 | Fungal Zn(2)-Cys(6) binuclear cluster domain / Fungal specific transcription factor domain | *FZC10* | |
| 151 | 841 | Fungal Zn(2)-Cys(6) binuclear cluster domain / Fungal specific transcription factor domain | *PZC40* | |
| 152 | 6223 | MIZ/SP-RING zinc finger | *MIZ1* | |
| 153 | 830 | Fungal Zn(2)-Cys(6) binuclear cluster domain / Fungal specific transcript ion factor domain | *FZC38* | |
| 154 | 1551 | Glucocorticoid receptor-like (DNA-binding domain)/ GATA zinc finger | *GAT201* | |
| 155 | 4908 | bZIP transcription factor / Basic region leucine zipper | *CLR4* | |

To analyze the functions of the transcription factors, the present inventors deleted 155 putative transcription factor genes out of 178 using homologous recombination. To perform a large-scale *in vivo* virulence test, dominant nourseothricin-resistance markers (NATs) containing a series of signature tags were employed.

The genotypes of all transcription factor mutant strains were confirmed by performing Southern blot analysis to verify both the gene deletion and the absence of any ectopic integration of each gene-disruption cassette in transcription factor mutant strains. To accurately validate the phenotype and exclude unlinked mutational effects, the present inventors generated more than two independent transcription factor mutants for all 155 transcription factors, including four known transcription factors (HXL1, ATF1, MBS1 and SKN7) (Non-Patent Document 9, Non-Patent Document 10 and Non-Patent Document 11), and thus obtained a total of 322 strains (see Table 2 below).

**[Table 2]**

| Transcription factor mutants | | | | |
|---|---|---|---|---|
| | H99 1D | Designated name | TF class | Strain in format in |
| 1 | 02566 | *FKH2* | FKH | YSB1339 |
| 2 | | | | YSB1340 |
| 3 | 00791 | *HLH1* | HLH | YSB1175 |
| 4 | | | | YSB1176 |
| 5 | 07464 | *MBS1* | APS | YSB488 |
| 6 | | | | YSB489 |
| 7 | 03401 | *GAT203* | GAT | YSB569 |
| 8 | | | | YSB570 |
| 9 | 04588 | *ERT1* | FZC | YSB693 |
| 10 | | | | YSB694 |
| 11 | 00828 | *SIP401* | FZC | YSB1358 |
| 12 | | | | YSB1359 |
| 13 | 03561 | *FZC33* | FZC | YSB1074 |
| 14 | | | | YSB1075 |
| 15 | 06762 | *GAT204* | GAT | YSB1311 |
| 16 | | | | YSB1312 |
| 17 | 06276 | *CEP3* | FZC | YSB847 |
| 18 | | | | YSB848 |
| 19 | 05785 | *SIB4* | FZC | YSB1013 |
| 20 | | | | YSB1014 |
| 21 | 01438 | *MBS2* | APS | YSB538 |
| 22 | | | | YSB539 |
| 23 | 07593 | *YAP4* | BZP | YSB1587 |
| 24 | | | | YSB1661 |
| 25 | 04837 | *MLN1* | HLH | YSB1172 |
| 26 | | | | YSB1173 |
| 27 | 05093 | *HOB6* | HOM | YSB1255 |
| 28 ' | | | | YSB1256 |
| 29 | 05642 | *FZC37* | FZC | YSB1329 |
| 30 | | | | YSB1330 |
| 31 | 05431 | *RIM101* | C2Z | YSB1366 |
| 32 | | | | YSB1367 |
| 33 | 04398 | *ARO80* | FZC | YSB714 |
| 34 | | | | YSB715 |
| 35 | 04878 | *FZC1* | FZC | YSB510 |
| 36 | | | | YSB511 |
| 37 | 03183 | *FZC24* | FZC | YSB774 |
| 38 | | | | YSB775 |
| 39 | 03710 | *ECM22* | FZC | YSB476 |
| 40 | | | | YSB478 |
| 41 | 03279 | *CCD4* | HOM | YSB706 |
| 42 | | | | YSB707 |
| 43 | 04637 | *MBF1* | HTH | YSB768 |
| 44 | | | | YSB769 |
| 45 | 06425 | *PPR1* | FZC | YSB1046 |
| 46 | | | | YSB1047 |
| 47 | 04345 | *AR08001* | FZC | YSB661 |
| 48 | | | | YSB662 |
| 49 | 04184 | *FZC47* | FZC | YSB1406 |
| 50 | | | | YSB1407 |
| 51 | 02774 | *MAL13* | FZC | YSB506 |
| 52 | | | | YSB507 |
| 53 | 00670 | *FZC12* | FZC | YSB467 |
| 54 | | | | YSB468 |
| 55 | 05010 | *ZFC7* | C2Z | YSB481 |
| 56 | | | | YSB482 |
| 57 | 04090 | *ATF1* | BZP | YSB676 |
| 58 | | | | YSB678 |
| 59 | 06134 | *BZP1(HXL1)* | BZP | YSB723 |
| 60 | | | | YSB724 |
| 61 | 04630 | *YAP2* | BZP | YSB1416 |
| 62 | | | | YSB1417 |
| 63 | 07901 | *FZC29* | FZC | YSB718 |
| 64 | | | | YSB719 |
| 65 | 03115 | *FZC46* | FZC | YSB1209 |
| 66 | | | | YSB1210 |
| 67 | 07924 | *MCM1* | SRF | YSB1302 |
| 68 | | | | YSB1303 |
| 69 | 07435 | *HAP2* | CCA | YSB1104 |
| 70 | | | | YSB1105 |
| 71 | 02555 | *SIP402* | FZC | YSB529 |
| 72 | | | | YSB530 |
| 73 | 04594 | *FZC27* | FZC | YSB582 |
| 74 | | | | YSB583 |
| 75 | 06188 | *FZC15* | FZC | YSB646 |
| 76 | | | | YSB647 |
| 77 | 05170 | *PIP2* | FZC | YSB 1249 |
| 78 | | | | YSB1250 |
| 79 | 02241 | *HOB5* | HOM | YSB1585 |
| 80 | | | | YSB1586 |
| 81 | 06921 | *HOB4* | HOM | YSB1435 |
| 82 | | | | YSB1437 |
| 83 | 05186 | *GRF1* | GRF | YSB796 |
| 84 | | | | YSB797 |
| 85 | 00039 | *ZFC6* | C2Z | YSB1953 |
| 86 | | | | YSB1954 |
| 87 | 01454 | *STE12* | C2Z | YSB1542 |
| 88 | | | | YSB1543 |
| 89 | 03527 | *HEL2* | C2Z | YSB1382 |
| 90 | | | | YSB1383 |
| 91 | 05255 | *FZC2* | FZC | YS81050 |
| 92 | | | | YSB1051 |
| 93 | 01883 | *GAT8* | GAT | YSB471 |
| 94 | | | | YSB472 |
| 95 | 04353 | *CLR1* | C2Z | YSB1396 |
| 96 | | | | YSB1397 |
| 97 | 03998 | *RLM1* | SRF | YSB1300 |
| 98 | | | | YSB1301 |
| 99 | 00239 | *YAP1* | BZP | YSB815 |
| 100 | | | | YSB1290 |
| 101 | 06871 | *FZC41* | FZC | YSB1334 |
| 102 | | | | YSB1335 |
| 103 | 00156 | *SP1(CRZ1)* | C2Z | YSB 1 263 |
| 104 | | | | YSB1264 |
| 105 | 04268 | *APN2* | GRF | YSB1429 |
| 106 | | | | YSB1430 |
| 107 | 05420 | *USV101* | C2Z | YSB1464 |
| 108 | | | | YSB1465 |
| 109 | 00018 | *FZC6* | FZC | YSB1980 |
| 110 | | | | YSB1981 |
| 111 | 03346 | *BZP4* | BZP | YSB1894 |
| 112 | | | | YSB1895 |
| 113 | 05538 | *JJJ1* | C2Z | YSB1592 |
| 114 | | | | YSB1594 |
| 115 | 03409 | *SKN7* | USE | YSB349 |
| 116 | | | | YSB350 |
| 117 | 06339 | *FZC35* | FZC | YSB1341 |
| 118 | | | | YSB1342 |
| 119 | 07506 | *FAP1* | NFX | YSB813 |
| 120 | | | | YSB817 |
| 121 | 04807 | *FZC8* | FZC | YSB2112 |
| 122 | | | | YSB2113 |
| 123 | 02435 | *BWC2* | GAT | YSB1839 |
| 124 | | | | YSB1840 |
| 125 | 02364 | *FZC19* | FZC | YSB2115 |
| 126 | | | | YSB2116 |
| 127 | 03116 | *HCM1* | FKH | YSB1850 |
| 128 | | | | YSB1851 |
| 129 | 02877 | *FZC51* | FZC | YSB1842 |
| 130 | | | | YSB1843 |
| 131 | 00559 | *BZP3* | BZP | YSB1099 |
| 132 | | | | YSB1100 |
| 133 | 03914 | *FZC14* | FZC | YSB1846 |
| 134 | | | | YSB1847 |
| 135 | 00871 | *CLR3* | BZP | YSB1834 |
| 136 | | | | YSB1836 |
| 137 | 06483 | *FZC25* | FZC | YSB518 |
| 138 | | | | YSB1822 |
| 139 | 07797 | *CRL6* | FKH | YSB1106 |
| 140 | | | | YSB1107 |
| 141 | 05019 | *FZC21* | FZC | YSB1252 |
| 142 | | | | YSB1253 |
| 143 | 05380 | *FZC44* | FZC | YSB2182 |
| 144 | | | | YSB2183 |
| 145 | 04518 | *ZFC5* | C2Z | YSB2177 |
| 146 | | | | YSB2178 |
| 147 | 05176 | *HOB3* | HOM | YSB2001 |
| 148 | | | | YSB2002 |
| 149 | 05861 | *FKH101* | FKH | YSB1855 |
| 150 | | | | YSB1856 |
| 151 | 00460 | *LIV1* | HLH | YSB2211 |
| 152 | | | | YSB2212 |
| 153 | 02305 | *FZC45* | FZC | YSB2221 |
| 154 | | | | VSB2222 |
| 155 | 03849 | *ASG1* | FZC | YSB3013 |
| 156 | | | | YSB3014 |
| 157 | 01014 | *ZFC4* | C2Z | YSB32231 |
| 158 | | | | YSB2232 |
| 159 | 01858 | *HOB2* | HOM | YSB2282 |
| 160 | | | | YSB2283 |
| 161 | 06719 | *FZC49* | FZC | YSB2171 |
| 162 | | | | YSB2173 |
| 163 | 04093 | *XRM103* | FZC | YSB2298 |
| 164 | | | | YSB2299 |
| 165 | 04176 | *HSF2* | HSF | YSB2295 |
| 166 | | | | YSB2296 |
| 167 | 01431 | *HOB1* | HOM | YSB2308 |
| 168 | | | | YSB2309 |
| 169 | 07443 | *HLH4* | HOM | YSB2244 |
| 170 | | | | YSB2245 |
| 171 | 04916 | *FZC16* | FZC | YSB2326 |
| 172 | | | | YSB2327 |
| 173 | 05392 | *ZAP104* | C2Z | YSB2134 |
| 174 | | | | YSB2135 |
| 175 | 02322 | *FZC17* | FZC | YSB2250 |
| 176 | | | | YSB2251 |
| 177 | 04012 | *FZC18* | FZC | YSB2320 |
| 178 | | | | YSB2321 |
| 179 | 00505 | *FZC28* | FZC | YSB2337 |
| 180 | | | | YSB2338 |
| 181 | 06751 | *HLH3* | HLH | YSB2329 |
| 182 | | | | VSB2330 |
| 183 | 04841 | *FZC43* | FZC | YSB517 |
| 184 | | | | YSB2334 |
| 185 | 03212 | *HCM101* | FKH | YSB2390 |
| 186 | | | | YSB2391 |
| 187 | 01626 | *ADA2* | MYB | YSB2381 |
| 188 | | | | YSB2382 |
| 189 | 03894 | *PDR802* | FZC | YSB2387 |
| 190 | | | | YSB2388 |
| 191 | 02066 | *FZC13* | FZC | YSB2517 |
| 192 | | | | YSB2518 |
| 193 | 06818 | *HAP1* | FZC | YSB2481 |
| 194 | | | | YSB2482 |
| 195 | 05940 | *ZFC3* | C2Z | YSB2108 |
| 196 | | | | YSB2386 |
| 197 | 01948 | *FZC36* | FZC | YSB2335 |
| 198 | | | | YSB2523 |
| 199 | 04804 | *SRE1* | HLH | YSB2493 |
| 200 | | | | YSB2.494 |
| 201 | 04352 | *ZAP103* | C2Z | YSB2540 |
| 202 | | | | YSB2541 |
| 203 | 03768 | *FZC32* | FZC | YSB2385 |
| 204 | | | | YSB2526 |
| 205 | 04895 | *FZC3* | FZC | YSB2611 |
| 206 | | | | YSB2664 |
| 207 | 04583 | *DDT1* | DDT | YSB1583 |
| 208 | | | | YSB2633 |
| 209 | 01973 | *ZFC2* | C2Z | YSB2622 |
| 210 | | | | YSB2623 |
| 211 | 00156 | *FZC7* | FZC | YSB2704 |
| 212 | | | | YSB2705 |
| 213 | 03431 | *FZC48* | FZC | YSB2646 |
| 214 | | | | YSB2647 |
| 215 | 07922 | *FZC4* | FZC | YSB2724 |
| 216 | | | | YSB2725 |
| 217 | 00031 | *MLR1* | FZC | YSB2727 |
| 218 | | | | YSB2728 |
| 219 | 03018 | *ASG101* | FZC | YSB2697 |
| 220 | | | | YSB2698 |
| 221 | 04263 | *BZP2* | BZP | YSB2702 |
| 222 | | | | YSB2703 |
| 223 | 01708 | *GAT7* | GAT | YSB2699 |
| 224 | | | | YSB2700 |
| 225 | 00332 | *SIP4* | FZC | YSB2680 |
| 226 | | | | YSB2681 |
| 227 | 07724 | *CUF1* | CDB | YSB2665 |
| 228 | | | | YSB2666 |
| 229 | 03902 | *RDS2* | FZC | YSB1898 |
| 230 | | | | YSB1899 |
| 231 | 03366 | *ZNF2* | C2Z | YSB2740 |
| 232 | | | | YSB2741 |
| 233 | 05222 | *NRG1* | C2Z | YSB3096 |
| 234 | | | | YSB3097 |
| 235 | 05049 | *PIP201* | FZC | YSB3099 |
| 236 | | | | YSB3100 |
| 237 | 02516 | *HLH5* | HLH | YSB2609 |
| 238 | | | | YSB3059 |
| 239 | 04774 | *FZC26* | FZC | YSB3084 |
| 240 | | | | YSB3085 |
| 241 | 03336 | *FZC50* | FZC | YSB3131 |
| 242 | | | | YSB3132 |
| 243 | 03086 | *FZC20* | FZC | YSB3128 |
| 244 | | | | YSB3129 |
| 245 | 03229 | *Y0X101* | HOM | YSB3134 |
| 246 | | | | YSB3136 |
| 247 | 01841 | *GLN3* | GAT | YSB3154 |
| 248 | | | | YSB3155 |
| 249 | 02476 | *YRM101* | FZC | YSB2997 |
| 250 | | | | YSB2998 |
| 251 | 05153 | *GAT5* | GAT | YSB3033 |
| 252 | | | | YSB3034 |
| 253 | 02700 | *ZFC8* | C2Z | YSB3()31, |
| 254 | | | | YSB3032 |
| 255 | 04586 | *HOB7* | HOM | YSB3026 |
| 256 | | | | YSB3027 |
| 257 | 02723 | *FZC23* | FZC | YSB3105 |
| 258 | | | | YSB3106 |
| 259 | 03741 | *FZC31* | FZC | YSB3093 |
| 260 | | | | YSB3094 |
| 261 | 04457 | *FZC30* | FZC | YSB2447 |
| 262 | | | | YSB2448 |
| 263 | 04836 | *FZC10* | FZC | YSB3083 |
| 264 | | | | YSB3368 |
| 265 | 06223 | *MIZ1* | MIZ | YSB2133 |
| 266 | | | | YSB3366 |
| 267 | 01551 | *GAT201* | GAT | YSB3300 |
| 268 | | | | YSB3301 |
| 269 | 04908 | *CLR4* | BZP | YSB3282 |
| 270 | | | | YSB3283 |
| 271 | 07940 | *BZP5* | BZP | YSB1474 |
| 272 | | | | YSB1475 |
| 273 | 05112 | *FZC42* | FZC | YSB687 |
| 274 | | | | YSB690 |
| 275 | 00193 | *GAT1* | GAT | YSB2972 |
| 276 | | | | YSB2973 |
| 277 | 06814 | *SXI1alpha* | HOM | YSB1390 |
| 278 | | | | YSB1391 |
| 279 | | | | YSB1392 |
| 280 | 00896 | *FZC34* | FZC | YSB501 |
| 281 | | | | YSB2979 |
| 282 | 07411 | *RUM1* | PHZ | YSB3164 |
| 283 | | | | YSB3747 |
| 284 | 00830 | *ZC38* | FZC | YSB777 |
| 235 | | | | YSB3791 |
| 286 | 01069 | *FZC11* | FZC | YSB845 |
| 287 | | | | YSB846 |
| 288 | | | | YSB2983 |
| 289 | 03132 | *FZC5* | FZC | YSB1400 |
| 290 | | | | YSB1401 |
| 291 | | | | YSB1404 |
| 292 | 00068 | *MET32* | C2Z | YSB1178 |
| 293 | | | | YSB1179 |
| 294 | | | | YSB1180 |
| 295 | 01173 | *PAN1* | P53 | YSB1181 |
| 296 | | | | YSB 1182 |
| 297 | | | | YSB1183 |
| 298 | 00514 | *GAT6* | GAT | YSB1384 |
| 299 | | | | YSB1385 |
| 300 | | | | YSB1386 |
| 301 | 07011 | *FZC22* | FZC | YSB1688 |
| 302 | | | | YSB1689 |
| 303 | | | | YSB2974 |
| 304 | 02603 | *ZFC1* | C2Z | YSB2573 |
| 305 | | | | YSB2574 |
| 306 | | | | YSB2575 |
| 307 | 04036 | *HSF3* | HSF | YSB2527 |
| 308 | | | | YSB2528 |
| 309 | | | | YSB2529 |
| 310 | 03059 | *FZC9* | FZC | YSB2984 |
| 311 | | | | YSB3266 |
| 312 | | | | YSB3267 |
| 313 | 06283 | *LIV4* | MYB | YS82089 |
| 314 | | | | YSB3755 |
| 315 | | | | YSB3756 |
| 316 | 00841 | *F7,C40* | FZC | YSB3088 |
| 317 | | | | YSB3758 |
| 318 | 01977 | *FZC39* | FZC | YSB1820 |
| 319 | | | | YSB2621 |
| 320 | 05375 | *HLH2* | HLH | YSB1147 |
| 321 | | | | YSB1148 |
| 322 | | | | YSB1149 |

For parallel *in vitro* and *in vivo* phenotypic analysis, the present inventors deleted 53 TF genes, which were previously deleted in the CMO18 strain (a less virulent H99 strain, Non-Patent Document 12), and derived more than two independent mutants. Certain known transcription factors, including *RIM101, ADA2, CUF1, SXL1, SP-1*/*CRZ1, NRG1, STE12, BWC2, SRE1, ZNF2* and *HAP1*/*HAP2,* were also independently deleted here to accurately compare phenotypes. When two independent transcription factor mutants showed inconsistent phenotypes, additional transcription factor mutants were generated to exclude outlier mutants. The present inventors found that about 8% of gene knockouts (13 transcription factors) exhibited inconsistent phenotypes, potentially attributable to unexpected alterations in the genome. This level (7%) was highly similar to that reported in a similar study on the ascomycete fungal pathogen *Candida albicans* (Non-Patent Document 13). For the remaining 23 transcription factors, transcription factor mutants were not generated. In summary, the present inventors constructed a *Cryptococcus neoformans* transcription factor mutant collection that covers 155 transcription factors and 322 transcription factor mutant strains in total.

Out of the 156 transcription factors whose mutants were constructed, 58 transcription factor genes possess names designated in published studies or reserved by other researchers through registration in FungiDB (www.fungidb.org). For the remaining 98 transcription factors, the present inventors provided gene names by following the systematic genetic nomenclature flowchart in *Cryptococcus neoformans* recently reported (Non-Patent Document 14).

### 1.2: Construction of Transcription Factor Mutants

*Cryptococcus neoformans* transcription factor knockout mutants (hereinafter referred to as TFKO) were constructed in the C. *neoformans* serotype A H99S strain background. Gene-disruption cassettes containing the nourseothricin-resistance marker (NAT) and signature-tagged sequences were generated by overlap polymerase chain reaction (hereinafter referred to as PCR) or double-joint PCR strategies using the primer sets shown in the Sequence List and Table 2 (Non-Patent Document 15 and Non-Patent Document 16). In the overlap PCR process, the 5'- and 3'-flanking regions of the transcription factor genes were amplified by using primers L1 and R1 and primers L2 and R2 (see Table 1), respectively, together with H99 genomic DNA in the first round of PCR. Primers M13Fe (M13 forward extended) and M13Re (M13 reverse extended) were used for amplifying the dominant selectable marker (NAT) containing unique signature-tagged sequences. In the second round of PCR, the TF gene-disruption cassettes were generated by means of overlap PCR performed using primers L1 and R2 and the first-round PCR products as templates. In the double-joint PCR method, the 5'- and 3'-flanking regions of the transcription factor genes were amplified using, respectively, the primer pairs L1/L2 and R1/R2 with H99 genomic DNA in the first round of PCR. The 5'- and 3'-regions of NAT-split markers were amplified using primers M13Fe and NSL and primers M13Re and NSR, respectively, together with pNATSTM (obtained from Joeseph Heitman' Laboratory at Duke University), which harbored unique signature-tagged sequences. The amplified gene-disruption cassettes were combined with 600 µg of gold microcarrier beads (0.6 um, Bio-Rad) and treated with 10 µL of 2.5M calcium chloride and 2 µL of 1M spermidine. The gold beads combined with the gene-disruption cassettes were introduced into the H99S strains (obtained from Joeseph Heitman' Laboratory at Duke University) using the biolistic transformation apparatus (Non-Patent Document 17). After 4 hours of culture required for recovery, the cells were scraped, transferred onto an yeast extract-peptone dextrose (hereinafter referred to as YPD) medium containing 100µg/ml nourseothricin, and then incubated at 30°C for 3 to 5 days. Stable nourseothricin-resistant transformants were screened by diagnostic PCR using the primer sets listed in Table 3 and the Sequence List.

All transcription factor mutant strains were deposited in the Korean Culture Collection of Microorganisms (KCCM) and the Center of Microbial Pathogenesis at Duke University in USA.

**[Table 3]**

| Construction of primer sets for genes | | |
|---|---|---|
| Gene name | Primer name | Detailed description of primers |
| *FZC5* | L1 | CNAG_03132 5' flanking region primer 1 |
| | L2 | CNAG_03132 5' flanking region primer 2 |
| | R1 | CNAG_03132 3' flanking region primer 1 |
| | R2 | CNAG_03132 3' flanking region primer 2 |
| | SO1 | CNAG_03132 diagnostic screening primer, pairing with B79 |
| | PO2 | CNAG_03132 Southern blot probe primer |
| | STM | NAT#5 STM primer |
| | STM common | STM common primer |

### Example 2: Phenotypic Profiling

For the 322 transcription factor mutants constructed, the present inventors performed a series of *in vivo* and *in vitro* phenotypic analyses for the phenotypic classes as follows: growth, differentiation, morphology, stress responses, antifungal drug resistance, virulence-factor production and *in vivo* virulence. This overall phenome data set is illustrated together with a color scale, and data for transcript levels of each transcription factor measured by RNA sequencing analyses under six distinct growth conditions were measured. Red and blue in a thermal map showed decrease and increase, respectively. Phenotype strengths (strong, intermediate and weak) are distinguished in gradients of red or blue. The phenotypic analysis revealed that about 93% of the transcription factor mutants (145/155) exhibited at least one discernable phenotype, suggesting a high functional coverage of this transcription factor mutant collection. 85% of the transcription factors (132/155) have not been functionally characterized before in *Cryptococcus neoformans* strains.

All of these phenome data are publicly available in the *Cryptococcus neoformans* transcription factor database (http://tf.cryptococcus.org).

### 2.1: Genotypic Analysis

The accuracy of the genotypes of the positive transformants was validated by means of Southern blot analysis. Cryptococcus genomic DNA was extracted using the CTAB (cetyl trimethyl ammonium bromide) method (Non-Patent Document 33). Isolated genomic DNA from each TFKO mutant was digested with the indicated restriction enzyme (see Table 4). The digested genomic DNAs were separated by 1% agarose gel electrophoresis. The agarose gel was transferred into the denatured buffer containing 0.5M NaOH and 1.5M NaCl and allowed to stand for 45min. Next, the agarose gel was transferred into the neutralization buffer containing 1.5M NaCl and 0.5M Tris buffer adjusted with pH 8 and allowed to stand for 45min. The digested genomic DNAs were transferred to the nylon membrane using 10x SSC (saline sodium citrate) buffer and fixed by 1,200J/m² ultraviolet exposure. The membrane was hybridized with a gene-specific and radioactively labeled probe using modified church hybridization buffer (1mM EDTA, 0.25M Na₂HPO₄, 1% hydrolysated casein, 7% SDS, 6% H₃PO₄). The membrane was washed for 15minutes with washing buffer 1 (containing 2x SSC and 0.1% SDS) and washing buffer 2 (containing 1x SSC and 0.1% SDS). Next, the membrane was exposed to autography film for 1 day.

**[Table 4]**

| List of transcription factor knockout mutants and restriction enzymes used | | | |
|---|---|---|---|
| | H99 ID | Designated name | Restriction enzyme cut |
| 1 | 02566 | *FKH2* | BamH I |
| 2 | 00791 | *HLH1* | BglII |
| 3 | 01069 | *FZC11* | SphI |
| 4 | 07464 | *MBS1* | Sphl |
| 5 | 03401 | *GAT203* | HindIII |
| 6 | 04588 | *ERT1* | EcoRV |
| 7 | 00828 | *SIP401* | EcoRI |
| 8 | 01561 | *FZC33* | EcoRV |
| 9 | 06762 | *GAT204* | KpnI |
| 10 | 06276 | *CEP3* | SphI |
| 11 | 05785 | *STB4* | BamHI |
| 12 | 03132 | *FZC5* | PstI |
| 13 | 01438 | *MBS2* | EcoRI |
| 14 | 07593 | *YAP4* | SmaI |
| 15 | 04837 | *LN1* | SmaI |
| 16 | 05093 | *HOB6* | KpnI |
| 17 | 05642 | *FZC37* | XmaI |
| 18 | 05431 | *RIM101* | Clal |
| 19 | 04398 | *AR080* | HincII |
| 20 | 04878 | *FZC1* | EcoRI |
| 21 | 03183 | *FZC24* | EcoRV |
| 22 | 03710 | *ECM22* | HindIII |
| 23 | 03279 | *CCD4* | HindIII |
| 24 | 04637 | *MBF1* | HindIII |
| 25 | 06425 | *PPR1* | EcoRV |
| 26 | 04345 | *AR08001* | BamHI |
| 27 | 04184 | *FZC47* | BglII |
| 28 | 02774 | *MAL13* | EcoRV |
| 29 | 00670 | *FZC12* | StyI |
| 30 | 00068 | *MET32* | Afel |
| 31 | 05010 | *ZFC7* | Seal |
| 32 | 04090 | *ATF1* | PstI |
| 33 | 06134 | *BZP1(HXL1)* | HamH I |
| 34 | 04630 | *YAP2* | HindIII |
| 35 | 07901 | *FZC29* | EcoRV |
| 36 | 01173 | *PAN1* | SalI |
| 37 | 03115 | *FZC46* | SacI |
| 38 | 07924 | *MCM1* | XbaI |
| 39 | 07435 | *HAP2* | SalI |
| 40 | 02555 | *SIP402* | MfeI |
| 41 | 04594 | *FZC27* | HindIII |
| 42 | 06188 | *FZC15* | BamHI |
| 43 | 05170 | *PIP2* | BamHI |
| 44 | 02241 | *HOB5* | Pst I |
| 45 | 06921 | *HOB4* | KpnI |
| 46 | 05186 | *GRF1* | EcoRI |
| 47 | 00896 | *FZC34* | HindIII |
| 48 | 00039 | *ZFC6* | KpnI |
| 49 | 07940 | *REP9* | BamHI |
| 50 | 06814 | *SXI1alpha* | Sad |
| 51 | 01454 | *STE12* | BanHI |
| 52 | 03527 | *HEL2* | BamHI |
| 53 | 05255 | *FZC2* | HindIII |
| 54 | 05112 | *FZC42* | BglII, EcoRV |
| 55 | 01883 | *GAT8* | SacI |
| 56 | 04353 | *CLR1* | Sall |
| 57 | 05375 | *HLH2* | HindIII |
| 58 | 03998 | *RLM1* | StyI |
| 59 | 00239 | *YAP1* | BamHI |
| 60 | 06871 | *FZC41* | BamHI, XbaI |
| 61 | 00156 | *SP1(CRZ1)* | EcoRV |
| 62 | 04268 | *APN2* | EcoRV |
| 63 | 05420 | *USV101* | SphI |
| 64 | 00018 | *FZC6* | SphI |
| 65 | 03346 | *BZP4* | PstI |
| 66 | 00514 | *GAT6* | EcoRV |
| 67 | 05538 | *JJJ1* | SphI |
| 68 | 03409 | *SKN7* | BamHI |
| 69 | 06339 | *FZC35* | HindIII |
| 70 | 07011 | *FZC22* | PstI |
| 71 | 07506 | *FAP1* | HindIII |
| 72 | 04807 | *FZC8* | PstI |
| 73 | 02435 | *BWC2* | HindIII |
| 74 | 02364 | *FZC19* | BamHI |
| 75 | 03116 | *HCM1* | SphI |
| 76 | 02877 | *FZC51* | SphI |
| 77 | 00559 | *BZP3* | Sphl |
| 78 | 03914 | *FZC14* | PstI |
| 79 | 00871 | *CLR3* | XbaI |
| 80 | 06483 | *FZC25* | PstI |
| 81 | 07797 | *CRL6* | EcoRI |
| 82 | 05019 | *FZC21* | PstI |
| 83 | 05380 | *FZC44* | SacI |
| 84 | 04518 | *ZFC5* | EcoRI |
| 85 | 05176 | *HOB3* | Xhol |
| 86 | 05861 | *FKH101* | EcoRV |
| 87 | 00460 | *LIV1* | SphI |
| 88 | 02305 | *FZC45* | EcoRV |
| 89 | 03849 | *ASG1* | KpnI |
| 90 | 01014 | *ZPC4* | Sail |
| 91 | 01858 | *HOB2* | SphI |
| 92 | 06719 | *FZC49* | PstI |
| 93 | 04093 | *YRM103* | SphI |
| 94 | 04176 | *HSF2* | KpnI |
| 95 | 01431 | *HOB1* | HindIII |
| 96 | 07443 | *HLH4* | SphI |
| 97 | 04916 | *FZC16* | EcoRI |
| 98 | 05392 | *ZAP104* | SalI |
| 99 | 02322 | *FZC17* | Xbal |
| 100 | 04012 | *FZC18* | HindIII |
| 1()1 | 00505 | *FZC28* | Sma I |
| 102 | 06751 | *HLH3* | SalI |
| 103 | 04841 | *FZC43* | Xbal |
| 104 | 03212 | *HCM101* | SacI, Sail |
| 105 | 01626 | *ADA2* | BamHI |
| 106 | 03894 | *PDR802* | Sph I |
| 107 | 02066 | *FZC13* | BamHI |
| 108 | 06818 | *HAP1* | HindIII |
| 109 | 05940 | *ZFC3* | EcoRI |
| 110 | 01948 | *FZC36* | XhoI |
| 111 | 04804 | *SRE1* | BamHI |
| 112 | 04352 | *ZAP103* | SacI, SalI |
| 113 | 03768 | *FZC32* | BamHI |
| 114 | 01977 | *FZC39* | KpnI |
| 115 | 04895 | *FZC3* | Kpnl |
| 116 | 04583 | *DDT1* | SphI |
| 117 | 01973 | *ZFC2* | SphI |
| 118 | 02603 | *ZFC1* | HindIII |
| 119 | 04036 | *HSF3* | SphI |
| 120 | 06156 | *FZC7* | EcoRI |
| 121 | 03431 | *FZC48* | EcoRV |
| 122 | 07922 | *FZC4* | EcoRV |
| 123 | 00031 | *MLR1* | PstI, XmaI |
| 124 | 03018 | *ASG101* | XhoI |
| 125 | 04263 | *BZP2* | EcoRV |
| 126 | 01708 | *GAT7* | SacI |
| 127 | 00332 | *SIP4* | PstI |
| 128 | 07724 | *CUF1* | KpnI |
| 129 | 03902 | *RDS2* | EcoRV, EcoRI |
| 130 | 03366 | *ZNF2* | EcoRV |
| 131 | 05222 | *NRG1* | EcoRV |
| 132 | 05049 | *PIP201* | BamHI |
| 133 | 02516 | *HLH5* | BamHI |
| 134 | 04774 | *FZC26* | Pst I |
| 135 | 03059 | *FZC9* | EcoRV |
| 136 | 00193 | *GAT1* | SphI |
| 137 | 03336 | *FZC50* | HindIII |
| 138 | 03086 | *FZC20* | KpnI |
| 139 | 03229 | *Y0X101* | XbaI |
| 140 | 01841 | *GLN3* | EcoRI |
| 141 | 02476 | *YRM101* | Xhol |
| 142 | 05153 | *GAT5* | SalI |
| 143 | 02700 | *ZFC8* | EcoRV |
| 144 | 4586 | *HOB7* | SphI |
| 145 | 2723 | *FZC23* | EcoRV |
| 146 | 374 1 | *FZC31* | ClaI |
| 147 | 4457 | *FZC30* | BamHI |
| 148 | 6283 | *LIV4* | EcoRV |
| 149 | 7411 | *RUM1* | EcoRV |
| 150 | 4836 | *FZC10* | EcoRV |
| 151 | 841 | *FZC40* | EcoRI |
| 152 | 6223 | *MIZ1* | E coRV |
| 153 | 830 | *FZC38* | Sphl |
| 154 | 1551 | *G4T201* | Xbal |
| 155 | 4908 | *CLR4* | *HindIII* |

### 2.2: Analysis of Gene Expression

Gene expression was analyzed by Northern blot analysis. Total RNA was extracted from each sample using Trizol reagent. 10 µg of the RNA was separated in 1% agarose gel made with DEPC (diethyl pyrocarbonate)-treated water and 1x MOPS (3-(N-morpholino)propane sulfonic acid) running buffer by electrophoresis. The gel was washed three times with distilled water, transferred to a nylon membrane (Millipore, INYC00010) using 20 x SSC buffer, and fixed by 1200 J/m² ultraviolet exposure. The membrane was hybridized with a gene-specific and radioactively labeled probe using modified church hybridization buffer (1 mM EDTA; Biosesang Co., Ltd,. E1002), 0.25M Na₂HP0₄ (Sigma, S9763), 1% N-2-Amine (Sigma, C0626), 7% SDS (Bioshop, SDS001) and 0.17% H₃PO₄ (Sigma, #438081)). The membrane was washed with washing buffer 1 (2x SSC and 0.1% SDS) and washing buffer 2 (1x SSC and 0.1% SDS). Next, the membrane was exposed to autography film for 1 to 2 days.

### Example 3: Transcription Factors (TFs) Governing Growth

*C. neoformans* undergoes both saprobic and pathogenic life cycles in natural and animal host environments. Therefore, it must be capable of growing at temperatures ranging from ambient temperature (25°C) to high temperature (37 to 39°C) . In order to analyze the growth phenotypes of the transcription factor mutants at various temperatures, the growth of each mutant on yeast extract-peptone dextrose (YPD) medium [yeast extract (Becton, Dickison and company #212750), peptone (Becton, Dickison and company #211677), glucose (Duchefa, #G0802)] at various temperatures (25°C, 30°C, 37°C and 39°C) was observed. Deletion of some transcription factors (BZP2, CUF1, LIV4, GAT5, FZC6 and NRG1) resulted in temperature-independent growth defects. The growth defect of the cuf1 mutant was due to its inability to uptake copper, because external addition of CuSO₄ restored its wild-type (WT) growth.

In the present invention, the growth-defect transcription factor mutants (24 mutants) were classified into two groups: (1) temperature-independent growth-defect transcription factor mutants; and (2) temperature-dependent growth-defect transcription factors. The first transcription factor group (temperature-independent) includes *BZP2, CUF1, HOB1, GAT5, FZC6* and *NRG1.* Deletion of the transcription factors of the second group, including *HXL1, CRX1, ATF1, ADA2, LIV4, AR080, USV101, FZC31, MLN1, FZC30, FZC1, MIZ1, FZC46, APN2, GAT6, MBS2, SRE1,* and *ERT1,* caused growth defects only at high temperature (37 to 39°C) . Among these, only *HXL1,* which is a transcription factor downstream of the Ire1 kinase in the UPR signaling pathway, exhibited a severe growth defect at host physiological temperature. By contrast, deletion of *MLN1, MCM1* and *FZC46* promoted the growth of C. *neoformans* at 39°C. Collectively, these results suggest that multiple transcription factors (27 transcription factors) control -both positively and negatively- the growth and thermotolerance of C. *neoformans.*

### Example 4: Transcription Factors Governing Mating

In a natural environment, *C. neoformans* exists mainly in the yeast form but undergoes either bisexual differentiation with cells of the opposite mating type or unisexual differentiation with cells of the same mating type to produce filamentous forms and generate infectious basidiospores. These developmental processes contribute to the generation of the genetic diversity of the pathogen (Non-Patent Document 17).

To analyze mating phenotypes, the present inventors set up unilateral mating crosses by co-culturing each TF mutant (the serotype A MAT strain) with serotype A MATa wild-type KN99a strain (obtained from Joeseph Heitman's laboratory at Duke University). Each strain was cultured in YPD medium at 30°C for 16hours, and equal concentration of cells (10⁷ cells per ml) were mixed, spotted onto V8 mating media (pH 5; per 1 L: 50 ml V8 juice (Campbell), 0.5 g KH₂PO₄ (Bioshop, PPM302), 40 g Agar (Bioshop, AGR001.500)) and incubated in a dark at room temperature for 1 to 2 weeks. Filamentous growth was monitored weekly and photographed using an Olympus BX51 microscope equipped with a SPOT Insight digital camera (Diagnostic Instrument Inc.).

To monitor the expression of pheromone gene, cell fusion assay and Northern blot analysis were performed. For the cell fusion assay, each MAT transcription factor mutant or control strain (YSB119; obtained from Joeseph Heitman's Laboratory at Duke University) containing NAT^{R} marker and MATa control strain (YSB121; obtained from Joeseph Heitman's Laboratory at Duke University) containing neomycin-resistant (Neo^{r}) marker were cultured at 30°C in liquid YPD medium for 16hours, and the concentration of cells was adjusted to 10⁷ cells per ml with distilled water. Each MAT strain and MATa strain were mixed in an equal volume, spotted onto V8 medium and incubated in a dark at room temperature for 24hours. Then, the cells were scraped, resuspended in 1ml distilled water and spread onto YPD medium containing both nourseothricin (100µg/ml) and G418 (50µg/ml, Geneticin, Life technologies). The plates were further incubated at 30°C and the number of colonies on each plate was determined. In monitoring of pheromone gene expression, the MAT and KN99a strains were mixed with equal concentration of cells (10⁸ cells per ml), spread onto the V8 medium and incubated in the dark at room temperature for 18 to 24hours. Then, cells were scraped from the V8 medium, pelleted at 4°C, frozen in liquid nitrogen, and lyophilized overnight. Total RNA was isolated using Trizol reagent according to the protocol described in the prior art document. The RNA was electrophoresed, and then transferred to a membrane. The membrane was hybridized with a mating pheromone gene (MF1)-specific probe amplified with primer B1894 (5'-TTTTACGCTTTTTGCAGATTCCGCCAAA-3'), B195 (5'-GACCACTGTTTCTTTCGTTCT-3') and JEC21 genomic DNA (genomic DNA extracted from the JEC21 strain).

To analyze mating phenotypes, the present inventors set up unilateral mating crosses by coculturing each transcription factor mutant with serotype A MATa KN99a strain. The novel mating-regulating transcription factors in the present invention, deletion of *BZP2, USV101, FZC1* and *ZAP104* severely reduced mating, even in unilateral matings, whereas deletion of *HLH1, HAP2* and *GAT1* highly enhanced mating efficiency. To determine the mating steps in which these transcription factors are involved, the present inventors measured the efficiency of cell fusion and pheromone production, which precede the filamentation step. The *bzp2, usv101, fzc1* and *zap104* mutants lacked the ability to engage in cell fusion with the MATa control strain and also failed to induce pheromone gene (MF1) expression upon mating. Such results suggest that *Bzp2, Usv101, Fzc1* and *Zap104* transcription factors promote pheromone gene expression, which results in a subsequent increase in cell fusion. Conversely, in the *hlh1, hap2* and *gat1* mutants, cell-fusion efficiency was increased two- to three-fold, and pheromone gene expression was highly enhanced. *SKN7,* whose deletion promoted mating, was dispensable for both pheromone gene expression and cell fusion, indicating that it is likely involved in a later stage of mating. Analysis performed according to the present invention suggested that 34 transcription factors are involved in mating.

### Example 5: Transcription Factors Modulating Virulence-Factor Production

To support survival and proliferation within the host, C. *neoformans* is armed with several virulence factors, which include capsule and melanin. Capsule is a glucuronoxylomannan- or galactoxylomannan-based polysaccharide that protects cells from being phagocytosed by host phagocytic cells (Non-Patent Document 18). Melanin, a black-brown pigment made of polyphenol complexes, confers both antiphagocytic and antioxidant activity to cells (Non-Patent Document 19).

### 5.1: Capsule Production

Capsule production was measured in both qualitative and quantitative manners as described in the prior art documents (Non-Patent Document 20 and Non-Patent Document 21). Cells were grown at 30°C in liquid YPD medium for 16hours, spotted onto Dulbecco's Modified Eagle's (DME) solid medium and incubated at 37°C for 2 days. Then, the cells were scraped from DME solid medium and washed with PBS (phosphate buffered saline). For qualitative measurement, capsules were stained by India ink (Bactidrop; Remel), and visualized using an Olympus BX51 microscope equipped with a Spot insight digital camera (Diagnostic Instrument Inc.). For quantitative measurement, the cells collected from DME solid medium were fixed with 10% formalin, and an equal number of cells (2.5 × 10⁷ cells per ml) were loaded into a haematocrit capillary tube, which was subsequently placed vertically to allow the cells to be packed by gravity for 10 days. The packed cell volume ratio was measured by calculating the ratio of the length of the packed cell volume phase to the length of the total volume phase (cells + medium). The relative packed cell volume of each mutant was measured by calculating the ratio of the mutant packed cell volume ratio to the wild-type packed cell volume ratio.

Triplicate technical experiments with two or more independent strains were performed. Statistical difference in relative packed cell volume was determined by one-way analysis of variance with Bonferroni's multiple-comparison test using Prism 6 (GraphPad software).

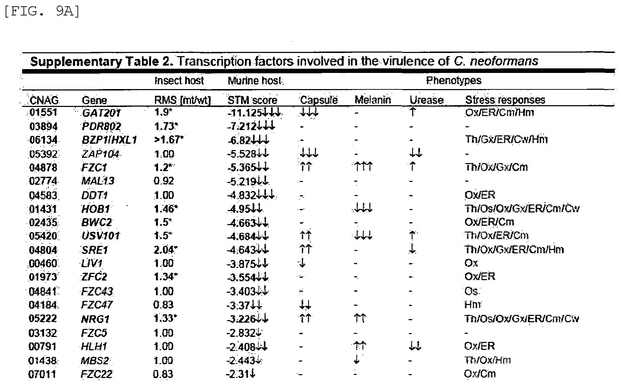

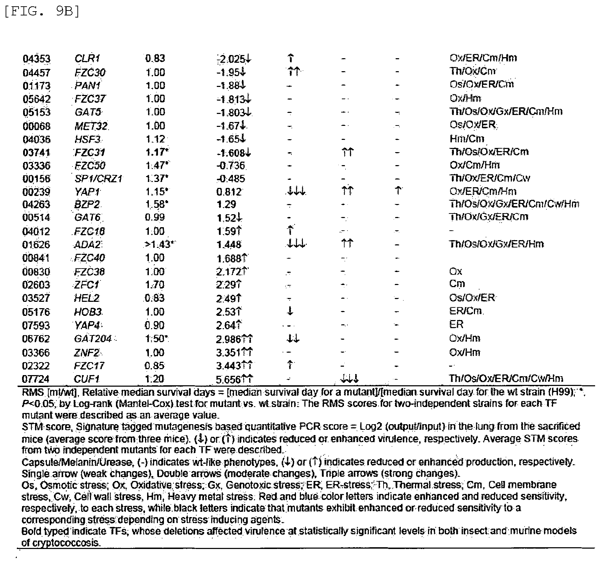

In the present invention, it was found that 49 transcription factors are involved in capsule production (FIG. 8a; 8b-29 negative regulators, 8c-20 positive regulators). Such transcription factors include previously reported capsule-regulating transcription factors, such as Atf1 (Non-Patent Document 10), Mbs1 (Non-Patent Document 11), Gat201 (Non-Patent Document 12) and Ada2 (Non-Patent Document 22). In addition to such capsule-regulating transcription factors, the present inventors identified several novel capsule-regulating transcription factors. The *zap104Δ, bap1Δ* and *rds2Δ* mutants also exhibited severely reduced capsule production compared to the results observed in the previously reported *gat201Δ* and *ada2Δ* mutants. Thus, Ada2, Gat201, Zap104, Bap1 and Rds2 together with 15 other positive regulators were predicted as major positive regulators in capsule production. By contrast, deletion of HOB7, CLR3 and FZC51 greatly enhanced capsule production, suggesting that these transcription factors together 26 other negative regulators are major negative regulators in capsule production.

### 5.2: Measurement of Melanin Production and Analysis of LAC1 Expression

Each transcription factor mutant strain and wild-type strain were cultured in liquid YPD medium at 30°C overnight, spotted on Niger seed agar medium containing 0.1% or 0.3% glucose, and then cultured at 37°C, and the plates were photographed daily to determine melanin production. The present inventors uncovered 27 transcription factors (11 positive regulators and 16 negative regulators) involved in melanin production. A few of transcription factors, including Cuf1, Stel2, Mbs1, Skn7 and Atf1, are previously reported transcription factors (Non-Patent Document 10, Non-Patent Document 11, Non-Patent Document 23, Non-Patent Document 24 and Non-Patent Document 25). In addition to such results, the *fzc8Δ, hob1Δ* and *bzp4Δ* mutants exhibited greatly reduced melanin production; the reduction was similar to that of the *cuf1Δ* mutant.

In addition, in the present invention, the correlation between transcription factors and the expression of LAC1, which is the major laccase involved in melanin synthesis, was examined. First, Northern blot analysis was performed to monitor the induction of LAC1. Each wild-type and each transcription mutant were cultured in YPD medium at 30°C for 16 hours. The cultured cells were adjusted to an OD₆₀₀ of 0.15 (optical density at 600 nm) and inoculated into 150 ml of fresh YPD liquid medium. The cell culture was further cultured at 30°C until it reached at an OD₆₀₀ of about 0.6. To prepare the zero-time sample, 50ml of 150ml cell culture was sampled and 100ml of the remaining culture was pelleted by centrifugation. After removal of the supernatant, the cells were re-suspended in glucose-free YNB liquid medium (Becton, Dickison and company, #291940). During culture, 50 ml of the cell culture was sampled at 1 hr and 2 hr. Total RNA was extracted from each sample, amplified by PCR using H99 genomic DNA, primer B3662 (5'-CTTTCAATCGTCCAAGCG-3') and primer B3663 (5'-CCCCAGTTATCCAAAAAGTC-3'), and subjected to Northern blot analysis using an LAC1-specific probe. As a result, the present inventors found that Hob1 and Fzc8 promote the expression of LAC1, which is the major laccase involved in melanin synthesis (Non-Patent Document 26), under glucose-starvation conditions, whereas Bzp4 and Cuf1 are not directly involved in LAC1 expression. By contrast, deletion of *HLH1, HLH2, BAP1* and *FZC1* greatly enhanced melanin production, although only Hlh1 negatively regulated LAC1 expression. Therefore, the present inventors identified novel positive regulators (Hob1 and Fzc45) and negative regulator (Hlh1) of *LAC1* in *Cryptococcus neoformans.*

### 5.3: Urease Production

In addition to capsule and melanin, crucial factors involved in the virulence of *Cryptococcus neoformans* include urease, a nickel-dependent protein complex (Ure1, Ure4, Ure6 and Ure7) that converts urea into ammonia, which serves as a nitrogen source.

Each wild-type strain and transcription factor mutant strain were cultured at 30°C in liquid YPD medium for 16 hours (overnight) and washed with distilled water, and then the cell density was adjusted to 1 × 10⁷ cells per ml. Next, 5 µl of the cells (5 × 10⁴ cells) were spotted onto Christensen's agar medium. Then, the cells were incubated for 7 to 10 days at 30°C and photographed during incubation.

The present inventors found that 19 transcription factors are involved in either positively (15 transcription factors) or negatively (4 transcription factors) regulating urease production.

### Example 6: Transcription Factors Affecting Infectivity and Virulence of C. neoformans

Identification of transcription factors required for the pathogenicity of C. *neoformans* is critical for future development of novel antifungal drugs and therapeutic methods. The present inventors employed two large-scale assays: (1) a virulence assay conducted in the invertebrate insect larval model system *Galleria mellonella;* and (2) a signature-tagged mutagenesis (STM)-based infectivity assay conducted in a murine inhalation model. These assays using one insect host and one mammalian host model have been widely adopted for large-scale virulence/infectivity assays in other fungi as well as C. *neoformans* (Non-Patent Document 12).

### 6.1: Insect-Based Virulence Assay

Insect-based virulence assay was performed using a modification of the previously known method (Non-Patent Document 21). For the insect-based virulence assay, 15 *Galleria mellonella* caterpillars (body weight: 250±50mg) in the final instar larval stage, reached within 7 days from the day of shipment (Vanderhorst Inc., St Marys, OH, USA), were randomly sorted into each group. Each C. *neoformans* strain was grown overnight at 30°C in YPD medium, washed three times, re-suspended with PBS, and used in hematocyte calculation performed using a hemocytometer. The present inventors inoculated 4,000 C. *neoformans* cells per larva through the second to last prolegs of larvae using a 100-µl Hamilton syringe equipped with a 10-µl-size needle and a repeating dispenser (PB600-1, Hamilton). As a non-infection control, PBS was injected. After injection, larvae were incubated in Petri dishes in humidified plastic containers and monitored daily. Infected larvae were incubated at 37°C and monitored daily. Larvae were considered dead when they displayed no movement when touched. Larvae that transformed into pupae during experiments were censored for statistical analysis. Survival curves were prepared using Prism 6 (GraphPad) and statistically analyzed using the Log-rank (Mantel-Cox) test. The present inventors first monitored the survival curve for a single mutant strain for each transcription factor gene (total 155) and statistically compared it with that of the wild-type strain. In the case of transcription factor mutant that showed statistically significant reduction or enhancement of virulence (P<0.05; Log-rank test) by gene deletion, the present inventors examined a second independent strain.

Each panel indicates virulence assay results for two independent mutants of each transcription factor. The identified mutants include nine transcription factor mutants *(hxl1, ada2, sre1, nrg1, bwc2, crz1, pdr802, gat201* and *gat204)* that were previously reported to show reduced virulence in a murine model of systemic cryptococcosis (Non-Patent Document 9, Non-Patent Document 12, Non-Patent Document 22, Non-Patent Document 27, Non-Patent Document 28, Non-Patent Document 29, Non-Patent Document 30, Non-Patent Document 31 and Non-Patent Document 32). This further indicated a strong correlation between the insect and murine models in terms of the pathogenicity of C. *neoformans.* Besides the deletion of these known TFs, deletion of *HOB1, BZP2, USV101, BAP1, ZFC2, FZC1, FZC50* and *FZC31* significantly reduced the virulence of C. *neoformans.* The present inventors identified 17 transcription factor genes involved in the virulence of *Cryptococcus neoformans* using the insect host model.

### 6.2: Animal Study

Animal care and all experiments were conducted in accordance with the ethical guidelines of the Institutional Animal Care and Use Committee (IACUC) of Yonsei University. The Yonsei University IACUC approved all of the vertebrate studies.

In the signature-tagged mutagenesis (STM)-based mouse infectivity test, transcription factor strains tagged with 44 distinct signature tags (Table 1) were grown at 30°C in YPD medium, washed three times with PBS and then pooled; the same number of cells of each strain were used after counting cells using a haemocytometer. The *ste50* and *ire1* mutants tagged with STM#282 and STM#169 sequences were used as virulent and non-virulent control strains, respectively (Non-Patent Document 9 and Non-Patent Document 33). Thus, the number of transcription factors (TFs) exhibiting the same signature tag is smaller than 4. In the present invention, four STM-based virulence tests were performed. To obtain the input TF genomic DNA library, the pooled TF mutants were 10-fold serially diluted, plated on YPD media, incubated at 30°C for 3 days and collected by scraping for use in isolating genomic DNA. The output TF genomic DNA library was obtained as follows. Seven-week-old female A/Jcr mice (Jackson Laboratory) anaesthetized with intraperitoneal injection of Avertin (2,2,2-tribromoethanol) were infected through intranasal inhalation of 5 × 10⁵ cells (in 50 µl volume) of the pooled TF mutants and sacrificed with an overdose of Avertin at 15 days post infection. For each set of assays, the present inventors used five mice. Two independent mutants for each TF were tested in a separate STM set assay. Mouse lungs were dissected and homogenized in PBS. Each lung-tissue lysate was spread on YPD media containing 100µg/ml of chloramphenicol, incubated at 30°C for 3 days, and then collected by scraping to isolate output genomic DNA. Both input and output genomic DNAs were extracted using the CTAB method (Non-Patent Document 21). Quantitative PCR analysis was performed using various tag-specific primers listed in Table S1 and a MyiQ2 Real-Time PCR detection system (Bio-Rad). The STM score (Log₂[output/input] was calculated according to the method described in the prior art documents (Non-Patent Document 12 and Non-Patent Document 34).

### 6.3: Results of Animal Study

Using the STM-based murine host model, the present inventors identified 40 virulence genes. The STM score for each mutant was calculated based on the quantitative PCR score=Log₂ (output/input) in the lung from the sacrificed mice (average score from three mice). Among all the sets studied, the *ire1Δ* mutant, which is a non-virulent control strain, exhibited a highly reduced STM score (-7.03±1.99), whereas the *ste50Δ* mutant, a virulent control strain, showed an STM score of 0.11±1.13. For supporting the quality of the STM assay, 11 of the 40 transcription factor genes identified in the present invention were previously reported to be involved in virulence (Non-Patent Document 9, Non-Patent Document 12, Non-Patent Document 29 and Non-Patent Document 30). The *gat201Δ* and *pdr802Δ* mutants exhibited drastically reduced STM scores (-11.125 and -7.212, respectively) compared to those described in the prior art document (Non-Patent Document 12). Similarly, the STM scores of the *zap104Δ* and *liv1Δ* mutants were also decreased (-5.528 and -3.875, respectively). However, the zap103Δ mutant showed a very high virulence as described in the prior art document (Non-Patent Document 12) (STM score =2.51). Furthermore, the *hxl1Δ, nrg1Δ* and *bwc2Δ* mutants also showed highly reduced STM scores. 11 of the 40 transcription factors identified by the STM analysis *(GAT201, PDR802, HXL1, BWC2, NRG1, FZC1, HOB1, USV101, ZFC2, SRE1* and *FZC31)* were also discovered using the insect model. The virulence assay data from the insect model were statistically significantly correlated with the STM-based infectivity data from the murine model based on the Pearson correlation coefficient (PCC) analysis. Among the 26 novel virulence-related transcription factors that were screened using only the STM-based murine model, the *fzc31Δ* and *ddt1Δ* mutants exhibited highly reduced STM scores (-4.328 and - 4.832, respectively). The phenome database according to the present invention revealed that the *fzc31Δ* mutant exhibited increased susceptibility to osmotic, oxidative and cell membrane stresses, which might collectively affect virulence. By contrast, the only notable phenotype observed in the case of the *ddt1Δ* mutant was a weak dithiothreitol (DTT) sensitivity, which is not likely responsible for the marked decrease in the survival of the mutant in the lung because several other DTT-sensitive TF mutants were as virulent as the WT strain.

### Example 7: Examination of Antifungal Drug Resistance and Susceptibility

For the treatment of cryptococcosis, amphotericin B (AmpB) with or without flucytosine (5-FC) and fluconazole (FCZ) are widely used (Non-Patent Document 2). However, in addition to the toxic side effects of such drugs, the emergence of antifungal drug-resistant Cryptococcus strains has caused serious clinical problems (Non-Patent Document 35). To identify any transcription factors involved in antifungal drug resistance, the present inventors monitored the alteration of antifungal drug susceptibility among the C. *neoformans* TFKO mutant strains.

Cells were grown at 30°C in liquid YPD medium for 16hours, 10-fold serially diluted (1 to 10⁴ dilutions), and spotted on YPD medium containing the indicated concentrations of the following antifungal drugs: fludiooxonil, fluconazole, amphotericin B, and flucytosine. The cells were incubated at 30°C and photographed for 2 to 5 days.

Numerous transcription factors were found to be involved in antifungal drug resistance, implying that Cryptococcus strains can potentially adapt to antifungal drugs in versatile manners. In response to FCZ, 35.5% of transcription factor mutants (55/155) exhibited either increased susceptibility (35 transcription factors) or resistance (20 transcription factors), suggesting that resistance to the azole-based antifungal drug could readily occur through the modulation of diverse transcription factors. However, in response to amphotericin B (AmpB), mutants of 56 genes exhibited differential susceptibility, with 47 transcription factor mutants showing increased susceptibility and only 8 transcription factor mutants exhibiting increased drug resistance. These data support the clinical observation that compared with azole resistance, polyene resistance is rarely observed. Furthermore, it is known that the 5-FC readily elicits the development of drug-resistant strains (Non-Patent Document 36). Supporting such results, the present inventors found that 27 transcription factors differentially regulate flucytosine resistance.

The present inventors noted that the deletion of some transcription factors negatively regulated azole and polyene susceptibility (Table 5 and FIG. 8a), possibly because these transcription factors might directly control *ERG11* expression and sterol biosynthesis and affect polyene-binding capacity. Two of these transcription factors were previously reported to be Erg11 regulators. Sre1, a key sterol regulatory transcription factor, forms a complex with Scp1 as a part of the sterol regulatory element-binding protein pathway in C. *neoformans* (Non-Patent Document 27 and Non-Patent Document 28). Mbs1 negatively regulates basal *ERG11* expression and therefore its deletion increases azole resistance but decreases polyene resistance in C. *neoformans* (Non-Patent Document 11). To further test whether other transcription factors are also involved in ERG11 regulation, the present inventors measured ERG11 expression levels in these TF mutants under both sterol-replete and sterol-depleted conditions (FIG. 8b). To analyze the expression of *ERG2, ERG3, ERG5, ERG11* and *ERG25,* the wild-type, *sre1* and *hob1* mutants were grown in liquid YPD medium overnight. The overnight culture was then inoculated in 100ml of fresh YPD medium at 30°C and grown until the OD₆₀₀ of the culture reached about 1.0. To prepare the zero-time sample, 50ml of cell culture was sampled, and the remaining culture was treated with fluconazole (final concentration: 10µg/ml) for 90min. Total RNA was isolated using TRIzol reagent, and cDNA was synthesized using M-MuLV reverse transcriptase (Thermo scientific). Northern blot analysis was performed with each ERG gene-specific probe that was amplified with ERG gene-specific primers with the total RNA in cells treated or not treated with fluconazole. Primers: B5789 (5'-CAAGAAATGGAGCGTGAG-3') and B5790 (5'-CAGTGTTGTAAAGCGTGATG-3') for ERG2; B1720 (5'-ATCCCTTTTCACCGTCGCTC-3') and B1721 (5'-CGTCGTGGATGAGAATAGTCC-3') for ERG3; B671 (5'-GTTTGTTGCCTGAGAACTGGG-3') and B674 (5'-ATCACTCAACTCGGTCCTCTCGTG-3') for ERG5; B678 (5'-TTCAGGGAACTTGGGAACAGC-3') and B1598 (5'-CAGGAGCAGAAACAAAGC-3') for ERG11; B1718 (5'-TGACCGCCTGTAGATTGTC-3') and B1719 (5'-TAGTCCCACCACCTGAAAC-3') for ERG25. Quantitative real-time PCR was performed with each gene-specific primer using a MyiQ2 Real-Time PCR detection system (Bio-Rad). Primers: B5789 (5'-CAAGAAATGGAGCGTGAG-3') and B6838 (5'-GGGAGGGTCGAGGATGTAGA-3') for ERG2; B1720 (5'-ATCCCTTTTCACCGTCGCTC-3') and B6838 (5'-GGGAGGGTCGAGGATGTAGA-3') for ERG3; B671 (5'-GTTTGTTGCCTGAGAACTGGG-3') and B672 (5'-GTAGATACTGAGAGCCTGCTTGGTG-3') for ERG5; B677 (5'-AATCTCCTTACCAGCCATTCGG-3') and B678 (5'-TTCAGGGAACTTGGGAACAGC-3') for ERG11; B2695 (5'-TCGTCTTTGGCAAGCAGTC-3') and B6840 ((5'-GAAGTCGTGGTGGTCAGCA-3') for ERG25; and B679 (5'-CGCCCTTGCTCCTTCTTCTATG-3') and B680 (5'-TACTCGTCGTATTCGCTCTTCG-3') for ACT1. As expected, basal and induced *ERG11* levels were substantially lower in the sre1 mutant than in the wild-type strain. Notably, deleting *HOB1* markedly increased the basal expression levels of *ERG11.* To determine whether Hob1 is involved in the regulation of other ERG genes, the present inventors monitored the expression of ERG2, ERG3, ERG5 and ERG25 in the wild-type strain, hob1Δ and sre1Δ strains under sterol-replete and -depleted conditions. The expression of all of these ERG genes was induced in response to sterol depletion through fluconazole treatment in the wild-strain strain, but not in the *sre1Δ* strain (FIG. 8c). Deletion of HOB1 markedly induced the basal expression of ERG2 (FIG. 8c). By contrast, under sterol depletion, the induction of *ERG2, ERG3, ERG5, ERG11* and *ERG25* was decreased in the *hob1Δ* mutant (FIG. 8c). The tight regulation of ERG expression appeared to be mostly absent in the *hob1Δ* mutant, indicating that Hob1 is a key regulator of ergosterol gene expression (FIG. 8d). Notably, the transcription factors involved in sterol biosynthesis also appeared to be involved in environmental stress responses and adaptation, which are critical for the survival and proliferation of C. *neoformans* within the host because the pathogen encounters drastic environmental changes during infection (Non-Patent Document 3).

**[Table 5]**

| Transcription factors involved in antifungal agent resistance in C. *neoformans* | | |
|---|---|---|
| Antifungal agents | TF mutants showing increased resistance | TF mutants showing increased suscept ibility |
| Azole (Fluconazole) | *HOB1, HAP2, SKN7, NRG1, MBS1, PPR1, JJJ1, HCM1, ADA2, FZC9, GAT7, ERT1, FKH2, ECM22, DDT1, GAT5, YRM103, CUF1, FZC51, LIV4,* | *BZP3, HLH3, BZP1*/*HXL1, SRE1, RIM101, YAP2, HLH1, YAP4, PIP2, MIZ1, MLN1, HOB6, MBF1, MET32, FZC46, YAP1, FZC14, FZC2, HSF2, ZFC6, FZC45, FZC30, ASG1, STE12, LIV1, FZC22, FZC31, PAN1, BZP2, SP1*/*CRZ1, BZP5, SXI1alpha, FZC34,* , *FZC17, HLH2* |
| Polyene (Amphotericin B) | *SRE1, YAP1, FZC51, SKN7, CLR1, BZP4, ATF1, FZC4* | *HOB1, MBS1, JJJ1, ERT1, ECM22, GAT201, ZAP104, SP1*/*CRZ1, FZC6, BZP5, HLH1, PIP2, HCM1, BZP2, USV101, HOB4, STE12, HOB5, GRF1, HEL2, FZC45, ASG1, FZC22, HOB6, PAN1, CUF1, FZC49, FZC1, BWC2, FAP1, FZC44, FZC8, FZC23, GAT204, NRG1, PIP201, RIM101, HLH3, BZP3, MLN1, MET32, ZFC2, FZC31, RUM1, PDR802, FZC10, HLH2* |
| 5-flucyotosine | *HLH3, RIM101, GAT204, HOB3, FZC50, ZNF2, RPS2, FZC31* | *NRG1, ZFC2, YAP1, MBS1, FZC6, YAP2, BZP3, JJJ1, HLH1, PIP2, APN2, FZC46, HAP2, FZC51, BZP5, HCM1, FZC19, BZP2, FZC44* |
| Phenylpyrrole Fungicide (Fludioxonil) | *NRG1, JJJ1, SP1*/*CRZ1, SKN7, GAT7, FAP1, ZFC2, GAT204, ZNF2, HEL2, FZC50, SRE1* | *USV101, ADA2, YAP1, FZC6, HLH1, PIP2, FZC46, H4P2, BZP1*/*HXL1, FKH2, LIV1, YAP2, BZP2, FZC21, HLH3, YRM101, BZP5, GLN3, ZFC8, DDT1, FZC22, HOB6, RLM1, MLN1, PAN1, FZC35, YRN103, ZFC3, ASG1, FZC41, FZC43, FZC51, H4P1, MET32, FZC32* |

### Example 8: Examination of Responses to External Stress

To analyze external stress-related phenotypes, cells were grown at 30°C in liquid YPD medium for 16hours, 10-fold serially diluted (1 to 10⁴ dilutions), and spotted on YPD medium containing the indicated concentrations of the following stress inducers: A: osmotic stress (sorbitol); B: oxidative stress [hydrogen peroxide (H₂O₂), tert-butyl hydroperoxide (TH) (an organic peroxide), menadione, diamide] ; C: endoplasmic reticulum (ER) stress [tunicamycin), DTT (dithiothreitol)]; D: genotoxic stress [methyl methanesulfonate (MMS), hydroxyurea (HU)]; E: cell membrane/wall-destabilizing stress [calcofluor white (CFW), Congo red (CR), and sodium dodecyl sulfate (SDS)]; F: heavy-metal stress (CdSO₄). Cells were incubated at 30°C and photographed for 2 to 5 days. The osmotic stress applied was a stress induced by any one selected from the group consisting of 1M to 1.5M sodium chloride (NaCl), 1M to 1.5M potassium chloride (KCl) and 2M sorbitol. It was shown that the antifungal agent-targeting genes against the osmotic stress induced by the sodium chloride were *ada2, aro8001, bap1, bzp2, fzcl3, fzcl9, fzc34, fzc42, fzc43, fzc51, gat5, gat7, hap2, hcm1, hob1, hob6, met32, pan1, rim101* and *skn7* genes, and the antifungal agent-targeting genes against the osmotic stress induced by the potassium chloride were *ada2, bzp2, bzp4, fzc32, fzc35, fzc44, fzc6, hap2, hob1, hob2, nrg1, rim101* and *yrm10.* It was shown that the antifungal agent-targeting genes against the osmotic stress induced by sorbitol were *bzp2, fzc6* and *hob1.*

The oxidative stress was examined by applying each of 2.5 mM to 3.5 mM hydrogen peroxide H₂O₂), 0.7 mM to 0.8 mM tert-butyl hydroperoxide (TH), 0.02 mM to 0.03 mM menadione, and 2 mM to 3 mM diamide (DA).

It was shown that antifungal agent-targeting genes against the oxidative stress induced by the hydrogen peroxide were *ada2, bapl, bzp2, bzp3, cuf1, fzcl3, fzc21, fzc22, fzc27, fzc31, fzc4, fzc46, fzc50, fzc9, gat1, gat201, gat204, gat5, hlh1, hob1, hob4, hob5, hob6, liv1, met32, miz1, nrg1, pan1, rim101, sip402, sp1(crz1), sre1, ste12* and *usv101,* and antifungal agent-targeting genes for the oxidative stress induced by the tert-butyl hydroperoxide were *ada2, asg1, atf1, bap1, bap2, bzp5, clr1, ecm22, fzc1, fzc15, fzc21, fzc31, fzc34, fzc44, fzc49, fzc51, fzc6, gat5, gat8, grf1, hcm1, hlh2, hob1, hob2, hob4, liv1, met32, mwc2, pan1, ppr1, rim101, rlm1, skn7, sre1, usv101, yrm103, zap103, zfc2* and *zfc4.* It was shown that antifungal agent-targeting genes for the oxidative stress induced by the menadione were *bap1, bzp2, ecm22, fzc26, fzc3, fzc34, fzc35, fzc37, fzc4, fzc44, fzc6, gat6, hel2, jjj1, nrg1* and *usv101,* and antifungal agent-targeting genes for the oxidative stress induced by the diamide were *bap1, bap2, bzp2, bzp5, fap1, fkh2, fzcl9, fzc21, fzc27, fzc3, fzc30, fzc31, fzc34, fzc38, fzc4, fzc46, fzc49, fzc8, gat5, gat6, hap2, hlh1, hlh3, hob1, hob6, hsf2, met32, miz1, mln1, pan1, pip2, rum1, sip402, sre1* and *zfc2.*

The endoplasmic reticulum (ER) stress was induced by each of 0.2 µg/ml to 0.3 µg/ml tunicamycin (TM) and 15 mM to 20 mM dithiothreitol (DTT), and as a result, it was shown that antifungal agent-targeting genes for the endoplasmic reticulum (ER) stress induced by the tunicamycin were *bzp1(hxl1), bzp3, fzc2, fzc21, fzc38, fzc40, fzc44, gat7, hlh1, liv4, met32, mln1, pip2, rim101, rlm1, sp1(crz1), sre1* and *ste12.* In addition, it was shown that antifungal agent-targeting genes for the endoplasmic reticulum (ER) stress induced by the dithiothreitol were *ada2, apn2, bzp1(hxl1), bzp2, clr1, cuf1, ddt1, fzc25, fzc31, gat201, gat5, hap2, hlh2, hob1, nrg1, rlm1, sre1* and *usv101.*

The genotoxic stress was induced by each of 0.03% to 0.06% methyl methanesulfonate (MMS) and 50 mM to 100 mM hydroxyurea (HU). As a result, it was shown that an antifungal agent-targeting gene for the genotoxic stress induced by the methyl methanesulfonate was any one gene selected from the group consisting of *apn2, bzp1(hxl1), bzp2, fzc1, fzc38, fzc4, fzc40, fzc6, gat5, gat6, hcm101, hob1, jjj1, miz1* and sre1, and the antifungal agent-targeting genes against the genotoxic stress induced by the hydroxyurea were *ada2, bzp1(hxl1), bzp2, fzc6, gat5, gat6, hcm1, hlh2, hob1, jjj1, mbs1, nrg1, skn7* and *sre1.*

The cell wall or cell membrane stress was induced by each of 3 mg/ml to 5 mg/mg calcofluor white (CFW), 0.8% to 1% Congo red (CR) and 0.03% sodium dodecyl sulfate (SDS). As a result, it was shown that an antifungal agent-targeting gene for the cell wall or cell membrane stress induced by the calcofluor white (CFW) was any one gene selected from the group consisting of *bap2, bzp1(hxl1), bzp2, hap2, hobl, nrg1, pip2, rim101* and *sp1(crz1),* and antifungal agent-targeting genes for the cell wall or cell membrane stress induced by the Congo red were *bap2, bzp1(hxl1), bzp2, cuf1, hap2, hlh3, hob1, nrg1, rim101* and *sp1(crz1),* and antifungal agent-targeting genes for the cell wall or cell membrane stress induced by the sodium dodecyl sulfate (SDS) were *alpha, asg1, asg101, bap1, bzp2, bzp3, bzp5, clr1, clr4, cuf1, ecm22, ert1, fap1, fzc21, fzc26, fzc30, fzc31, fzc7, gat1, gat201, gat5, gat6, gat7, hap2, hob1, hob3, hob5, jjj1, nrg1, pan1, pip2, rds2, rlm1, rum1, sip4, sp1(crz1), sre1, sxi1, usv101, zfc4* and *zfc6.*

The heavy-metal stress was induced by 20 µM to 30 µM cadmium sulfate (CdSO₄), and as a result, it was shown that antifungal agent-targeting genes for the stress induced by the heavy-metal stress were *aro80, aro8001, bzp2, bzp4, ccd4, cuf1, fap1, fzc10, fzc19, fzc35, fzc37, fzc46, fzc47, fzc51, fzc6, fzc8, gat5, gln3, hap2, hcm1, hob5, hob6, hob7, mbs2, mln1, pip2, pip201, rum1, skn7, yox101, yrm101* and *zfc8.*

Reflecting diverse types of external stresses, 145 transcription factors were identified to be involved in sensing and responding to at least one type of stress. Among these transcription factors, the two sterol regulators, Sre1 and Hob1, appeared to be general stress-responsive transcription factors that govern multiple stress responses and adaptations. Strikingly, the deletion of HOB1 or SRE1 substantially reduced resistance to osmotic/salt, oxidizing/reducing, genotoxic, endoplasmic reticulum (ER) and cell wall/membrane stresses. The hob1 and sre1 mutants exhibited similar stress resistance and susceptibility patterns under most of the various environmental stresses, and this is in stark contrast to their opposite resistance patterns towards fluconazole (FCZ) and amphotericin B (AmpB). This result strongly suggested that sterol homeostasis is a very important factor in controlling stress response and adaptation in C. *neoformans.*

### Example 9: Analysis of Functional Correlation

To understand the correlation among phenotypic traits and virulence, the present inventors measured the degree of linear dependence by calculating all Pearson Correlation Coefficients (PCCs) between two possible *in vitro* and *in vivo* phenotypic combinations tested in the present invention. These correlations are illustrated in a combined network. The correlation network revealed that the virulence of C. *neoformans* is highly correlated to growth at distinct temperatures and osmotic and cell wall/membrane-stress responses and moderately related to oxidative, genotoxic and ER stress responses. Notably, these stress phenotypes were also highly inter-correlated, suggesting that several core stress signaling networks might exist, including the known Hog1, Pkc1/Mpk1 and UPR signaling pathways. By contrast, mating and resistance to antifungal drugs, except to resistance to AmpB, were not significantly related to the virulence of C*. neoformans.* The production of virulence factors did not appear to be correlated to *in vivo* virulence, and this is likely because increased virulence-factor production often did not result in increased virulence. Supporting this, reduced capsule production was found to be highly correlated to reduced virulence by PCC analysis (P<0.05).

### Example 10: Construction of C. neoformans Transcription Factor Database

The genome and transcriptome data collected for 155 transcription factors were processed using the protocol of the standardized genome data in Comparative Fungal Genomics Platform (CFGP 2.0; Non-Patent Document 37).For detailed information of the predicted genes, pre-computed results of eight bioinformatics programs (InterPro scan, Signalp 3.0, PSortII, TargetP, ChloroP, SecretomeP, predictsNLS and TMHMM2) were provided (Non-Patent Document 38, Non-Patent Document 39, Non-Patent Document 40, Non-Patent Document 41, Non-Patent Document 42, Non-Patent Document 43, Non-Patent Document 44 and Non-Patent Document 45).

To browse genomics contexts together with key biological features, Seoul National University Genome Browser (SNUGB; Non-Patent Document 46) was incorporated for use with the *Cryptococcus* Transcription Factor Database. In the pages of Browse Scaffolds, Browse Gene Models and 3 gene-family browsers, direct links to the SNUGB module were provided. MySQL 5.0.81 (source code distribution) and PHP 5.2.6 were used for administrating the database and developing web interfaces, respectively. Web pages were provided through Apache 2.2.9 web server.

### Example 11: Construction of Pearson's Correlation Networks

The present inventors calculated Pearson Correlation Coefficient (PCC) PCC scores by using Prism 5.0 (GraphPad Software Inc.) based on the results of phenotypic tests (strongly resistant phenotype: 3; moderately resistant phenotype: 2; weakly resistant phenotype: 1; wild type-like phenotype: 0; weakly sensitive phenotype: -1; moderately sensitive phenotype: -2; and strongly sensitive phenotype: -3). Networks were visualized using Cytoscape software 3.2.0 based on the PCC scores.

### Accession Number

Name of Depositary Institution: Korean Culture Center of Microorganisms;
Accession Number: KCCM51291;
Date of Deposit: March 23, 2015.

### [Industrial Applicability]

The present invention relates to novel genes that regulate the virulence of *Cryptococcus neoformans* strains, and to the use thereof. According to the present invention, a novel antifungal agent and/or a novel agent for treating meningitis can be screened.

### General Deposition Number Notice for Microorganism

| No. 2015-21 | |
|---|---|
| For the microorganism to which you applied for general deposition under number 2015-21 on March 27, 2015, the general deposit was accepted, and the general deposit number of the microorganism is notified as follows. | |

| Follows | |
|---|---|
| 1. Name of microorganism deposited | *Cryptococcus neoformans var. grubil* H99S and transcription factor mutant library |
| 2. Name of microorganism at listing | |
| 3. Microorganism deposit number | KCCM 51291 |
| 4. Date of deposit | March 23, 2015 |

◊The above microorganism can be distributed without restrictions to researchers both in Korea and abroad for academic and industrial purposes.

Korean Culture Center of Microorganisms

## Claims

1. A method for screening an antifungal agent, comprising the steps of:
(a) bringing a sample to be analyzed into contact with a *Cryptococcus neoformans* cell containing a virulence regulatory gene which regulates melanin production;
(b) measuring expression of the virulence regulatory gene in the cell; and
(c) determining that the sample is an antifungal agent, when the expression of the virulence regulatory gene is down-regulated,
wherein the virulence regulatory gene is bzp4.

2. The method of claim 1, wherein additionally one or more genes selected from the group consisting of ert1, fzc25, fzc5, fzc8, hobl, liv1, mbs2 and usv101 is down-regulated.

3. The method of claim 1, wherein additionally one or more genes selected from the group consisting of ada2, bap1, bzp2, bzp3, fkh2, fzc1, fzc31, gat1, hlh1, hlh2, nrg1*,* rds2, sip4 and sip401 is up-regulated.

4. The method of any one of claims 1 to 3, wherein the cell in step (a) is any one cell selected from a cell line library deposited under accession number KCCM51291.

## Patentansprüche

1. Verfahren zum Screening eines antimykotischen Mittels, umfassend die Schritte, dass:
(a) eine zu analysierende Probe mit einer Zelle von *Cryptococcus neoformans* in Kontakt gebracht wird, die ein die Virulenz regulierendes Gen enthält, welches die Melanin-Produktion reguliert;
(b) die Expression des die Virulenz regulierenden Gens in der Zelle gemessen wird; und
(c) bestimmt wird, dass die Probe ein antimykotisches Mittel ist, wenn die Expression des die Virulenz regulierenden Gens herunterreguliert wird,
wobei das die Virulenz regulierende Gen bzp4 ist.

2. Verfahren nach Anspruch 1, wobei zusätzlich ein oder mehrere Gene ausgewählt aus der Gruppe bestehend aus ert1, fzc25, fzc5, fzc8, hob1, liv1, mbs2 und usv101 herunterreguliert werden.

3. Verfahren nach Anspruch 1, wobei zusätzlich ein oder mehrere Gene ausgewählt aus der Gruppe bestehend aus ada2, bap1, bzp2, bzp3, fkh2, fzc1, fzc31, gat1, hlh1, h1h2, nrg1, rds2, sip4 und sip401 hochreguliert werden.

4. Verfahren nach einem der Ansprüche 1 bis 3, wobei die Zelle in Schritt (a) eine beliebige Zelle ist, die aus einer Zelllinien-Bibliothek, die unter der Zugriffsnummer KCCM51291 hinterlegt ist, ausgewählt ist.

## Revendications

1. Procédé de criblage d'un agent antifongique, comprenant les étapes consistant à :
(a) mettre en contact un échantillon à analyser avec une cellule de *Cryptococcus neoformans* contenant un gène régulateur de virulence qui régule la production de mélanine ;
(b) mesurer l'expression du gène régulateur de virulence dans la cellule ; et
(c) déterminer que l'échantillon est un agent antifongique lorsque l'expression du gène régulateur de virulence est régulée à la baisse ;
dans lequel le gène régulateur de virulence est bzp4.

2. Procédé selon la revendication 1, dans lequel un ou plusieurs gènes choisis dans le groupe constitué par ert1, fzc25, fzc5, fzc8, hob1, liv1, mbs2 et usv101 sont en outre régulés à la baisse.

3. Procédé selon la revendication 1, dans lequel un ou plusieurs gènes choisis dans le groupe constitué par ada2, bap1, bzp2, bzp3, fkh2, fzc1, fzc31, gat1, hlh1, h1h2, nrg1, rds2, sip4 et sip401 sont en outre régulés à la hausse.

4. Procédé selon l'une quelconque des revendications 1 à 3, dans lequel la cellule dans l'étape (a) est une cellule quelconque choisie dans une bibliothèque de lignées cellulaires déposée sous le numéro d'accès KCCM51291.
